# FASCICULE DE BREVET EUROPEEN

(11) **EP 0 991 425 B1**
(45) Date de publication et mention de la délivrance du brevet: **09.03.2005**
(21) Numéro de dépôt: 98935066.5
(22) Date de dépôt: 30.06.1998
(51) Int. Cl.: A61K 48/00

(54) **AMELIORATION DU TRANSFERT D'ACIDE NUCLEIQUE DANS LES CELLULES D'ORGANISMES EUCARYOTES PLURICELLULAIRES ET COMBINAISON PERMETTANT LA MISE EN OEUVRE DU PROCEDE**
VERBESSERUNG DER VERABREICHUNG DER NUKLEINSÄURE IN ZELLEN DER PLURIZELLULÄREN EUKARYOTISCHEN ORGANISMEN UND KOMBINATION ZUR DURCHFÜHRUNG DES VERFAHRENS
PRO

(30) Priorité: 30.06.1997 FR 9708232; 01.12.1997 US 67487 P
(43) Date de publication de la demande: 12.04.2000
(73) Titulaire: INSTITUT GUSTAVE ROUSSY, 94805 Villejuif Cédex (FR); CENTRE NATIONAL DE LA RECHERCHE SCIENTIFIQUE (CNRS), 75016 Paris (FR)
(72) Inventeur: BUREAU, Michel, F-92210 Saint Cloud (FR); MIR, Lluis, F-91370 Verrières le Buisson (FR); SCHERMAN, Daniel, F-75012 Paris (FR)
(74) Mandataire: Warcoin, Jacques
(86) Numéro de dépôt international: PCT/FR1998/001399
(87) Numéro de publication internationale: WO 1999/001157

(56) Documents cités:
- WO-A-95/06129
- WO-A-96/14836
- WO-A-97/07826
- WO-A-98/43702
- HELLER ET AL: "IN VIVO GENE ELECTROINJECTION AND EXPRESSION IN RAT LIVER" FEBS LETTERS, vol. 389, 1996, pages 225-228, XP002058383 cité dans la demande
- NISHI ET AL: "HIGH-EFFICIENCY IN VIVO GENE TRANSFER USING INTRAARTERIAL PLASMID DNA INJECTION FOLLOWING IN VIVO ELECTROPORATION" CANCER RESEARCH, vol. 56, 1996, pages 1050-1055, XP002058384
- MIR L.M. ET AL: "Electrochemotherapy potentiation of antitumour effect of bleomycin by local electric pulses." EUR.J.CANCER, vol. 27, no. 1, 1991, pages 68-72,
- OSHIMA Y. ET AL: "Targeted gene transfert to corneal endothelium in vivo by elctric pulses." GENE THERAPY, vol. 5, 1998,

## Description

La présente invention se rapporte à une amélioration très remarquable du transfert in vivo d'acides nucléiques dans les cellules d'organismes eucaryotes pluricellulaires ou d'acides nucléiques associés à des produits permettant d'augmenter le rendement de tels transferts en utilisant des champs électriques faibles compris entre 1 et 600 V/cm, et à la combinaison d'un acide nucléique et du procédé de transfert selon l'invention pour leur utilisation en thérapie génique.

Le transfert de gènes dans une cellule donnée est à la base de la thérapie génique. Cependant, l'un des problèmes est de parvenir à faire pénétrer une quantité d'acide nucléique suffisante dans des cellules de l'hôte à traiter ; en effet, cet acide nucléique, en général un gène d'intérêt, doit être exprimé dans des cellules transfectées. L'une des approches retenue à cet égard a été l'intégration de l'acide nucléique dans des vecteurs viraux, en particulier dans des rétrovirus, des adénovirus

ou des virus associés aux adénovirus. Ces systèmes mettent à profit les mécanismes de pénétration cellulaire développés par les virus, ainsi que leur protection contre la dégradation. Cependant, cette approche présente des inconvénients, et en particulier un risque de production de particules virales infectieuses susceptibles de dissémination dans l'organisme hôte, et, dans le cas des vecteurs rétroviraux, un risque de mutagénèse insertionnelle. De plus, la capacité d'insertion d'un gène thérapeutique ou vaccinal dans un génome viral demeure restreinte.

En tout état de cause, le développement de vecteurs viraux utilisables en thérapie génique impose d'avoir recours à des techniques complexes de virus défectifs et de lignées cellulaires de complémentation.

Une autre approche (Wolf et al. Science 247, 1465-68, 1990 ; Davis et al. Proc. Natl. Acad. Sci. USA 93, 7213-18, 1996) a donc consisté à administrer dans le muscle ou dans la circulation un acide nucléique de nature plasmidique, associé ou non à des composés destinés à favoriser sa transfection, comme des protéines, des liposomes, des lipides chargés ou des polymères cationiques tels que le polyéthylènimine, qui sont de bons agents de transfection in vitro (Behr et al. Proc. Natl. Acad. Sci. USA 86, 6982-6, 1989 ; Felgner et al. Proc. Natl. Acad. Sci. USA 84, 7413-7, 1987 ; Boussif et al. Proc. Natl. Acad. Sci. USA 92, 7297-301, 1995).

En ce qui concerne le muscle, depuis la publication initale de J.A. Wolff et al. montrant la capacité du tissu musculaire à incorporer de l'ADN-injecté sous forme de plasmide libre (Wolff et, al. Science 247, 1465-1468, 1990) de nombreux auteurs ont tenté d'améliorer ce processus (Manthorpe et al., 1993, Human Gene Ther. 4, 419-431 ; Wolff et al., 1991, BioTechniques 11, 474-485). Quelques tendances se dégagent de ces essais, comme notamment :
- l'utilisation de solutions mécaniques pour forcer l'entrée de l'ADN dans les cellules, en adsorbant l'ADN sur des billes propulsées ensuite sur les tissus (« gene gun ») (Sanders Williams et al., 1991, Proc. Natl. Acad. Sci. USA 88, 2726-2730 ; Fynan et al., 1993, BioTechniques 11, 474-485). Ces procédés se sont avérés efficaces dans des stratégies de vaccination, mais ne touchent que les couches superficielles des tissus. Dans le cas du muscle, leur utilisation nécessiterait un abord chirurgical pour permettre d'accéder au muscle, car les particules ne traversent pas les tissus cutanés ;
- l'injection d'ADN, non plus sous forme de plasmide libre, mais associé à des molécules susceptibles de servir de véhicule facilitant l'entrée des complexes dans les cellules. Les lipides cationiques, utilisés dans de nombreux autres procédés de transfection, se sont avérés jusqu'à l'heure actuelle décevants, car ceux qui ont été testés se sont montrés inhibiteurs de la transfection (Schwartz et al., 1996, Gene Ther. 3, 405-411). Il en est de même pour les peptides et polymères cationiques (Manthorpe et al., 1993, Human Gene Ther. 4, 419-431). Le seul cas d'association favorable semble être le mélange polyvinylalcool ou polyvinylpyrrolidone avec l'ADN. L'augmentation résultant de ces associations ne représente qu'un facteur inférieur à 10 par rapport à l'ADN injecté nu (Mumper et al., 1996, Pharmaceutical Research 13, 701-709) ;
- le prétraitement du tissu à injecter avec des solutions destinées à améliorer la diffusion et/ou la stabilité de l'ADN (Davis et al., 1993, Hum. Gene Ther. 4, 151-159), ou à favoriser l'entrée des acides nucléiques, par exemple l'induction de phénomènes de multiplication ou de régénération de cellules. Les traitements ont concerné en particulier l'utilisation d'anesthésiques locaux ou de cardiotoxine, de vasoconstricteurs, d'endotoxine ou d'autres molécules (Manthorpe et al., 1993, Human Gene Ther. 4, 419-431 ; Danko et al., 1994, Gene Ther. 1, 114-121; Vitadello et al., 1994, Hum. Gene Ther. 5,11-18). Ces protocoles de prétraitement sont difficiles à gérer, la bupivacaïne en particulier nécessitant pour être efficace d'être injectée à des doses très proches des doses létales. La préinjection de sucrose hyperosmotique, destinée à améliorer la diffusion, n'augmente pas le niveau de la transfection dans le muscle (Davise et al., 1993).

D'autres tissus ont été transfectés in vivo soit en utilisant l'ADN plasmidique seul, soit en association avec des vecteurs synthétiques (revues de Cotten et Wagner (1994), Current Opinion in Biotechnology 4, 705; Gao et Huang (1995), Gene Therapy, 2, 710; Ledley (1995), Human Gene Therapy 6, 1129). Les principaux tissus étudiés ont été le foie, l'épithélium respiratoire, la paroi des vaisseaux, le système nerveux central et les tumeurs. Dans tous ces tissus, les niveaux d'expression des transgènes se sont révélés trop faibles pour entrevoir une application thérapeutique (par exemple au niveau du foie, Chao et aL (1996) Human Gene Therapy 7, 901), bien que certains résultats encourageants aient été récemment présentés pour le tranfert d'ADN plasmidique dans la paroi vasculaire (Iires et al. (1996) Human Gene Therapy 7, 959 et 989). Dans le cerveau, l'efficacité de transfert est très faible, de même que dans les tumeurs (Schwartz et al. 1996, Gene Therapy 3,405; Lu et al. 1994,Cancer Gene Therapy 1, 245; Son et al. Proc. Natl. Acad. Sci.USA 91,12669)

L'électroporation, ou utilisation de champs électriques pour perméabiliser des cellules, est également utilisée in vitro pour favoriser la transfection d'ADN dans des cellules en culture. Toutefois, il était jusqu'à présent admis que ce phénomène répondait à un effet dépendant d'un seuil et que cette électroperméabilisation ne pouvait être observée que pour des champs électriques d'intensité relativement élevée, de l'ordre de 800 à 1 200 Volts/cm pour les cellules animales. Cette technique a également été proposée in vivo pour améliorer l'efficacité d'agents antitumoraux, comme la bléomycine, dans des tumeurs solides chez l'homme (brevet américain n° 5 468 228, L.M. Mir). Avec des impulsions de très courte durée (100 microsecondes), ces conditions électriques (800 à 1 200 Volts/cm) sont très bien adaptées au transfert intracellulaire de petites molécules. Ces conditions (impulsions de 100 microsecondes) ont été appliquées sans amélioration pour le transfert d'acides nucléiques in vivo dans le foie, où des champs inférieurs à 1 000 Volts/cm se sont révélés totalement inefficaces, et même inhibiteurs par rapport à l'injection d'ADN en l'absence d'impulsions électriques (brevet WO 97/07826 et Heller et al. FEBS Letters, 389, 225-8, 1996).

Le document Nishi et al. (vol. 56, Cancer Res., 1996, pages 1050-1055) décrit que l'application du champ électrique est effectuée avant l'administration à la cellule tumorale du vecteur plasmidique dans l'expérience décrite. Nishi et al. précise que, selon les données antérieures, la prise par les cellules ayant subi l'électroporation à travers les pores membranaires n'est pas affectée par le fait que les molécules (acide nucléique) soient présentes pendant ou après l'impulsion d'électroporation (cf. page 1051, colonne de gauche, par, 2, lignes 19 à 22).

Cette technique présente d'ailleurs des difficultés d'application in vivo, car l'administration de champs d'une telle intensité peut provoquer des lésions tissulaires plus ou moins étendues, qui ne représentent pas un problème pour le traitement de patients cancéreux mais qui peuvent représenter un inconvénient majeur pour le sujet sain ou pour le sujet malade lorsque l'acide nucléique est administré dans des tissus autres que les tissus tumoraux.

Alors que toutes les études citées mentionnent la nécessité de champs électriques élevés, de l'ordre de 1 000 Volts/cm, pour être efficace in vivo, de manière vraiment inattendue et remarquable, les demandeurs ont à présent montré que le transfert d'acides nucléiques dans des tissus in vivo pouvait être augmenté de façon très importante, sans effets indésirables, en soumettant le tissu à des impulsions électriques d'intensité faible, par exemple de 100 ou de 200 Volts/cm, et d'une durée relativement longue. De plus, les demandeurs ont constaté que la grande variabilité d'expression du transgène observée dans l'art antérieur de transfert d'ADN était notablement réduite par le procédé selon l'invention.

C'est pourquoi, la présente invention concerne un procédé de transfert d'acides nucléiques in vivo, dans lequel les cellules des tissus sont mises en contact avec l'acide nucléique à transférer, par administration directe dans le tissu ou par administration topique ou systémique, et dans lequel le transfert est assuré par application auxdits tissus d'une ou de plusieurs impulsions électriques d'une intensité comprise entre 1 et 600 Volts/cm.

Selon un mode préféré le procédé selon l'invention s'applique à des tissus dont les cellules ont des géométries particulières comme par exemple des cellules de grande taille et/ ou de forme allongée et/ou répondant naturellement à des potentiels d'action électriques et/ ou ayant une morphologie spécifique.

De préférence, l'intensité du champ est comprise entre 200 et 600 Volts/cm et la durée totale d'application est supérieure à 10 millisecondes. Le nombre d'impulsions utilisées est par exemple de 1 à 100 000 impulsions et la fréquence des impulsions est comprise entre 0,1 et 1000 Hertz. De préférence la fréquence des impulsions est comprise entre 0,2 et 100 Hertz Les impulsions peuvent être aussi délivrées de manière irrégulière et la fonction qui décrit l'intensité du champ en fonction du temps peut être variable. A titre d'exemple, le champ électrique délivré peut résulter de la combinaison d'au moins un champ d'une intensité > à 400 V/cm et de préférence comprise entre 500 et 800 Volts/cm, de durée unitaire courte (< 1 msec), suivi de une ou plusieurs impulsions d'intensité plus faible , par exemple < 400 Volts/cm, et de préférence < 200 Volts /cm et de durée unitaire plus longue ( > 1 msec). L'intégrale de la fonction décrivant la variation du champ électrique avec le temps est supérieure à 1 kVxmsec/cm. Selon un mode préféré de l'invention, cette intégrale est supérieure ou égale à 5 kVxmsec/cm.

Selon un mode préféré de l'invention, l'intensité de champ des impulsions est d'environ 500 Volts/cm (*i.e.* ± 10 % et de préférence ± 5 %).

Les impulsions électriques sont choisies parmis les impulsions à ondes carrées, les champs électriques générant des ondes à décroissance exponentielle, des ondes unipolaires oscillantes de durée limitée, des ondes bipolaires oscillantes de durée limitée, ou d'autres formes d'ondes. Selon un mode préféré de l'invention, les impulsions électriques sont des impulsions à ondes carrées.

L'administration d'impulsions électriques peut être réalisée par toute méthode connue de l'homme du métier, par exemple :
- système d'électrodes externes placées de part et d'autre du tissu à traiter, notamment électrodes non invasives placées au contact de la peau,
- système d'électrodes implantées dans les tissus,
- système d'électrodes/injecteur permettant l'administration simultanée des acides nucléiques et du champ électrique.

Dans le cadre de la présente invention les termes transfert d'ADN ou d'acides nucléiques par application d'une ou plusieurs impulsions électriques, ainsi que les termes électrotransfert ou encore électrotransfection doivent être considérés comme équivalents et désignent le transfert d'acides nucléiques ou d'ADN par application ou en présence d'un champ électrique.

L'administration étant réalisée in vivo, il est parfois nécessaire d'avoir recours à des produits intermédiaires assurant la continuité électrique avec des électrodes externes non invasives. Il s'agira par exemple d'électrolyte sous forme de gel.

Les acides nucléiques peuvent être administrés par tout moyen approprié, mais sont de préférence injectés in vivo directement dans les tissus ou administrés par une autre voie, locale ou systémique et notamment au moyen d'un cathéter, qui les rend disponibles à l'endroit d'application du champ électrique. Les acides nucléiques peuvent être administrés avec des agents permettant ou facilitant le transfert, comme cela a été mentionné précédemment. Notamment, ces acides nucléiques peuvent être libres en solution ou associés à des agents synthétiques, ou portés par des vecteurs viraux. Les agents synthétiques peuvent être des lipides ou des polymères connus de l'homme du métier, ou bien encore des éléments de ciblage permettant la fixation sur la membrane des tissus cibles. Parmi ces éléments, on peut citer des vecteurs portant des sucres, des peptides, des anticorps ou des récepteurs hormonaux.

On conçoit, dans ces conditions de l'invention, que l'administration des acides nucléiques puisse précéder, être simultanée ou même suivre l'application des champs électriques.

C'est pourquoi, la présente invention a également pour objet un acide nucléique et un système produisant un champ électrique d'une intensité comprise entre 1 et 600 Volts/cm, comme produit de combinaison pour leur administration simultanée, séparée ou étalée dans le temps, aux cellules de mammifères et en particulier aux cellules humaines, in vivo. De préférence, l'intensité du champ est comprise entre 200 et 600 Volts/cm et, de manière encore plus préférée, l'intensité du champ est d'environ 500 Volts/cm.

Le procédé selon la présente invention est utilisable dans la thérapie génique, c'est-à-dire la thérapie dans laquelle l'expression d'un gène transféré, mais également la modulation ou le blocage d'un gène, permet d'assurer le traitement d'une pathologie particulière.

De préférence, les cellules des tissus sont traitées dans le but d'une thérapie génique permettant :
- soit la correction des dysfonctionnements des cellules elles-mêmes (par exemple pour le traitement des maladies liées à des déficiences génétiques comme par exemple la mucoviscidose),
- soit la sauvegarde et/ou la régénération de la vascularisation ou de l'innervation des tissus ou organes par des facteurs trophiques, neurotrophiques et angiogéniques produits par le transgène,
- soit la transformation du tissu en organe sécréteur de produits conduisant à un effet thérapeutique tels que le produit du gène lui-même (par exemple facteurs de régulation de thrombose et d'hémostase, facteurs trophiques, hormones) ou tels qu'un métabolite actif synthétisé dans le tissu grâce à l'adjonction du gène thérapeutique,
- soit une application vaccinale ou immunostimulante.

Un autre objet de l'invention est l'association des impulsions électriques d'un champ à des compositions contenant les acides nucléiques formulées en vue de toute administration permettant d'accéder au tissu par voie topique, cutanée, orale, vaginale, parentérale, intranasale, intraveineuse, intramusculaire, sous-cutanée, intra-oculaire, transdermique, etc. De préférence, les compositions pharmaceutiques de l'invention contiennent un véhicule pharmaceutiquement acceptable pour une formulation injectable, notamment pour une injection directe au niveau de l'organe désiré, ou pour toute autre administration. Il peut s'agir en particulier de solutions stériles, isotoniques, ou de compositions sèches, notamment lyophilisées, qui, par addition selon le cas d'eau stérilisée ou de sérum physiologique, permettent la constitution de solutés injectables. Les doses d'acide nucléique utilisées pour l'injection ainsi que le nombre d'administrations et le volume des injections peuvent être adaptées en fonction de différents paramètres, et notamment en fonction du mode d'administration utilisé, de la pathologie concernée, du gène à exprimer, ou encore de la durée du traitement recherchée.

Les acides nucléiques peuvent être d'origine synthétique ou biosynthétique, ou extraits de virus ou de cellules procaryotes ou de cellules eucaryotes provenant d'organismes unicellulaires (par exemple, levures) ou pluricellulaires. Ils peuvent être administrés en association de tout ou partie des composants de l'organisme d'origine et/ou du système de synthèse.

L'acide nucléique peut être un acide désoxyribonucléique ou un acide ribonucléique. Il peut s'agir de séquences d'origine naturelle ou artificielle, et notamment d'ADN génomique, d'ADNc, d'ARNm, d'ARNt et d'ARNr, de séquences hybrides ou de séquences synthétiques ou semisynthétiques d'oligonucléotides modifiés ou non. Ces acides nucléiques peuvent être obtenus par toute technique connue de l'homme du métier, et notamment par ciblage de banques, par synthèse chimique ou encore par des méthodes mixtes incluant la modification chimique ou enzymatique de séquences obtenues par ciblage de banques. Ils peuvent être modifiés chimiquement.

En particulier, l'acide nucléique peut être un ADN ou un ARN sens ou antisens ou à propriété catalytique comme un ribozyme. Par « antisens », on entend un acide nucléique ayant une séquence complémentaire à une séquence cible, par exemple une séquence d'ARNm dont on cherche à bloquer l'expression par hybridation sur la séquence cible. Par « sens », on entend un acide nucléique ayant une séquence homologue ou identique à une séquence cible, par exemple une séquence qui se lie à un facteur de transcription protéique et impliqué dans l'expression d'un gène donné. Selon un mode de réalisation préféré, l'acide nucléique comporte un gène d'intérêt et des éléments permettant l'expression dudit gène d'intérêt. Avantageusement, le fragment d'acide nucléique est sous forme d'un plasmide.

Les acides désoxyribonucléiques peuvent être simple ou double brin, de même que des oligonucléotides courts ou des séquences plus longues. Ils peuvent porter des gènes thérapeutiques, des séquences régulatrices de la transcription ou de la réplication, ou des régions de liaison à d'autres composants cellulaires, etc. Au sens de l'invention, on entend par « gène thérapeutique » notamment tout gène codant pour un ARN ou pour un produit protéique ayant un effet thérapeutique. Le produit protéique codé peut être une protéine, un peptide, etc. Ce produit protéique peut être homologue vis-à-vis de la cellule cible (c'est-à-dire un produit qui est normalement exprimé dans la cellule cible lorsque celle-ci ne présente aucune pathologie). Dans ce cas, l'expression du transgène permet par exemple de pallier à une expression insuffisante dans la cellule ou à l'expression d'une protéine inactive ou faiblement active en raison d'une modification, ou permet encore de surexprimer ladite protéine. Le gène thérapeutique peut aussi coder pour un mutant d'une protéine cellulaire, ayant une stabilité accrue, une activité modifiée, etc. Le produit protéique peut également être hétérologue vis-à-vis de la cellule cible. Dans ce cas, une protéine exprimée peut par exemple compléter ou apporter une activité déficiente dans la cellule (traitement des déficits enzymatiques), ou permettre de lutter contre une pathologie, ou stimuler une réponse immunitaire par exemple pour le traitement des tumeurs.Il peut s'agir d'un gène suicide (Thymidine Kinase de l'Herpès) pour le traitement des cancers ou de la resténose.

Parmi les produits thérapeutiques au sens de la présente invention; on peut citer plus particulièrement les gènes codant pour
- les enzymes, comme l'α-1-antitrypsine, les proteinase (métalloproteinases, urokinase, uPA, tPA,...streptokinase), les protéases clivant des précurseurs pour libérer des produits actifs (ACE, ICE,...), ou leurs antagonistes (TIMP-1, tissue plasminogen activator inhibitor PAI, TFPI
- les dérivés sanguins comme les facteurs impliqués dans la coagulation : facteurs VII, VIII, IX, les facteurs du complement, la thrombine,
- les hormones, ou les enzymes impliquées dans la voie de synthèse des hormones, ou les facteurs impliqués dans le contrôle de la synthèse ou de l'excrétion ou de la sécrétion des hormones, telles que l'insuline, les facteurs proches de l'insuline (IGF), ou l'hormone de croissance, l'ACTH, les enzymes de synthèse des hormones sexuelles,
- les lymphokines et cytokines : interleukines, chemokines (CXC et CC), interférons, TNF, TGF, facteurs chimiotactiques ou activateurs comme MIF, MAF, PAF, MCP-1, l'eotaxine, LIF, etc. (brevet français n° 92 03120),
- les facteurs de croissance, par exemple les IGF, EGF, FGF, KGF, NGF, PDGF, PIGF, HGF, proliferin
- les facteurs angiogéniques tels que les VEGF ou FGF, angiopoietine 1 ou 2, l'endotheline
- les enzymes de synthèse de neurotransmetteurs,
- les facteurs trophiques, en particulier neurotrophiques pour le traitement des maladies neurodégénératives, des traumatismes ayant endommagé le système nerveux, ou des dégénerescences rétiniennes, tels que les membres de la famille des neurotrophines tels que le NGF, BDNF, NT3, NT4/5, NT6 leurs dérivés et gènes apparentés - les membres de la familles du CNTF tels que le CNTF, l'axokine, le LIF et leurs dérivés - l'IL6 et ses dérivés - la cardiotrophine et ses dérivés - le GDNF et ses dérivés - les membres de la famille des IGF, tels que l'IGF-1, l'IFGF-2 et leurs dérivés
- les membres de la famille de FGF, tels que le FGF 1, 2, 3, 4, 5, 6, 7, 8, 9 et leurs dérivés, le TGFβ
- les facteurs de croissance osseuse,
- les facteurs hématopoïétiques, comme erythropoietine, les GM-CSF, M-CSF, LIF, etc.,
- les protéines de l'architecture cellulaire comme la dystrophine ou une minidystrophine (brevet français n° 91 11947), , les gènes suicides (thymidine kinase, cytosine déaminase, enzymes à cytochrome P450), les gènes de l'hémoglobine ou d'autres transporteurs protéiques,
- les gènes correspondant aux protéines impliquées dans le métabolisme des lipides, de type apolipoprotéine choisie parmi les apolipoprotéines A-I, A-II, A-IV, B, C-I, C-II, C-III, D, E, F, G, H, J et apo(a), les enzymes du métabolisme comme par exemple les lipases, la lipoprotéine lipase, la lipase hépatique, la lécithine cholestérol acyltransférase, la 7 alpha cholestérol hydroxylase, la phosphatidyl acide phosphatase,
ou encore des protéines de transfert de lipides comme la protéine de transfert des esters de cholestérol et la protéine de transfert des phospholipides, une protéine de liaison des HDL ou encore un récepteur choisi par exemple parmi les récepteurs aux LDL, les récepteurs des chylomicrons-remnants et les récepteurs scavenger, etc. On peut, de plus, ajouter la leptine pour le traitement de l'obésité.
- les facteurs régulant la pression sanguine, comme les enzymes impliquées dans le métabolisme du NO, l'angiotensine, la bradykinine, vasopressine, l'ACE, la rénine, les enzymes codant pour les mécanismes de synthèse ou de relargage des prostaglandines, du thromboxane, ou de l'adenosine, les récepteurs de l'adénosine, les kallikreines et kallistatines, ANP, ANF, les facteurs diurétiques ou antidiurétiques, les facteurs impliqués dans la synthèse, le métabolisme ou le relargage des médiateurs comme l'histamine, la sérotonine, les cathécholamines, les neuropeptides,
- les facteurs anti-angiogéniques comme le ligand de Tie-1 et de Tie-2, l'angiostatine, le facteur ATF, les dérivés du plasminogène, l'endothéline, les thrombospondines 1 et 2, le PF-4, l'interféron α ou β, l'interleukine 12, le TNFα, le récepteur de l'urokinase, fltl, KDR, PAI1, PAI2, TIMP1, le fragment prolactine
- les facteurs protégeant contre l'apoptose, comme la famille AKT,
- les protéines susceptible d'induire une mort cellulaire, soit actives par elles-mêmes comme les caspases, soit de type "pro-drogues" nécessitant une activation par d'autres facteurs, soit les protéines activant des pro-drogues en agent provoquant une mort cellulaire, comme la thymidine kinase du virus herpétique, les désaminase, permettant en particulier d'envisager des thérapies anti-cancéreures,
- les protéines impliquées dans les contacts et l'adhésion inter-cellulaires : VCAM, PECAM, ELAM, ICAM, intégrines, cathenines,
- les protéines de la matrice extra-cellulaire,
- les protéines impliquées dans la migration des cellules
- les protéines de type transduction du signal, type FAK, MEKK, p38 kinase, tyrosines kinases, serines- threonines kinases,
- les protéines impliquées dans la régulation du cycle cellulaire (p21, p16, cyclines, ...) ainsi que les protéines mutantes ou dérivées dominant négatif bloquant le cycle cellulaire et pouvant le cas échéant induire l'apoptose.
- les facteurs de transcription : jun, fos, AP1, p53,...et les protéines dela cascade de signalisation de p53.
- les protéines de structure de la cellule, comme les filaments intermédiaires (vimentine, desmine, kératines), la dystrophine, les protéines impliquées dans la contractilité et le contrôle de la contractibilité musculaire, en particulier les protéines impliquées dans le métabolisme calcique et les flux de calcium dans les cellules (SERCA, ...).
Dans les cas de protéines fonctionnant par des systèmes ligand et récepteurs, il est envisagable d'utiliser le ligand ou le récepteur (ex. FGF-R, VEGF-R, ...). On peut également citer des gènes codant pour des fragments ou des mutants de protéines de ligands ou de récepteurs, notamment des protéines précitées, présentant soit une activité supérieure à la protéine entière, soit une activité antagoniste, voire même de type " dominant négatif" par rapport à la protéine initiale (par exemple fragments de récepteurs inhibant la disponibilité de protéines circulantes, associés ou non avec des séquences induisant une sécrétion de ces fragments par rapport à un ancrage dans la membrane cellulaire, ou d'autres systèmes de modification du trafic intracellulaire de ces systèmes ligand- récepteurs de façon à détourner la disponibilité d'un des éléments) soit même possédant une activité propre distincte de celle de la protéine totale (ex. ATF).

Parmi les autres protéines ou peptides pouvant être sécrétés par le tissu, il est important de souligner les anticorps, les fragments variables d'anticorps simple chaîne (ScFv) ou tout autre fragment d'anticorps possédant des capacités de reconnaissance pour son utilisation en immunothérapie, par exemple pour le traitement des maladies infectieuses, des tumeurs, des maladies auto-immunes telles que la sclérose en plaques (anticorps antiidiotype), ainsi que les ScFv se fixant sur les cytokines pro-inflammatoires telles que par exemple IL1 et TNFα pour le traitement de l'arthrite rhumatoïde. D'autres protéines d'intérêt sont, de façon non limitative, des récepteurs solubles, comme par exemple le récepteur CD4 soluble ou le récepteur soluble du TNF pour la thérapie anti-HIV, le récepteur TNFα ou le récepteur soluble IL1 pour le traitement de l'arthrite rhumatoïde, le récepteur soluble de l'acétylcholine pour le traitement de la myasthénie ; des peptides substrats ou inhibiteurs d'enzymes, ou bien des peptides agonistes ou antagonistes de récepteurs ou de protéines d'adhésion comme par exemple pour le traitement de l'asthme, de la thrombose de la resténose, des métastases ou de l'inflammation ; des protéines artificielles, chimériques ou tronquées. Parmi les hormones d'intérêt essentiel, on peut citer l'insuline dans le cas du diabète, l'hormone de croissance et la calcitonine. On peut citer encore des protéines capables d'induire une immunité antitumorale ou stimuler la réponse immunitaire (IL2, GM-CSF, IL12, etc.). Enfin on peut citer les cytokines qui diminuent la réponse T_{H1} telles que IL10, IL4 et IL13.

Les nombreux exemples qui précèdent et ceux qui suivent illustrent l'étendue potentielle du champ d'application de la présente invention.

L'acide nucléique thérapeutique peut également être un gène ou une séquence antisens, dont l'expression dans la cellule cible permet de contrôler l'expression de gènes ou la transcription d'ARNm cellulaires. De telles séquences peuvent, par exemple, être transcrites dans la cellule cible en ARN complémentaire d'ARNm cellulaires et bloquer ainsi leur traduction en protéine, selon la technique décrite dans le brevet européen n° 140 308. Les gènes thérapeutiques comprennent également les séquences codant pour des ribozymes, qui sont capables de détruire sélectivement des ARN cibles (brevet européen n° 321 201).

Comme indiqué plus haut, l'acide nucléique peut également comporter un ou plusieurs gènes codant pour un peptide antigénique, capable de générer chez l'homme ou l'animal une réponse immunitaire. Dans ce mode particulier de mise en oeuvre, l'invention permet donc la réalisation soit de vaccins, soit de traitements immunothérapeutiques appliqués à l'homme ou à l'animal, notamment contre des microorganismes, des virus ou des cancers. Il peut s'agir notamment de peptides antigéniques spécifiques du virus d'Epstein Barr, du virus HIV, du virus de l'hépatite B (brevet européen n° 185 573), du virus de la pseudo-rage, du « syncitia forming virus », d'autres virus ou encore d'antigènes spécifiques de tumeurs comme les protéines MAGE (brevet européen n° 259 212), telles que les protéines MAGE 1, MAGE 2, ou des antigènes pouvant stimuler une réponse anti-tumorale telles que des protéines heat shock bactériennes.

Préférentiellement, l'acide nucléique comprend également des séquences permettant et/ou favorisant l'expression dans le tissu du gène thérapeutique et/ou du gène codant pour le peptide antigénique. Il peut s'agir des séquences qui sont naturellement responsables de l'expression du gène considéré lorsque ces séquences sont susceptibles de fonctionner dans la cellule transfectée. Il peut également s'agir de séquences d'origine différente (responsables de l'expression d'autres protéines, ou même synthétiques). Notamment, il peut s'agir de séquences promotrices de gènes eucaryotes ou viraux. Par exemple, il peut s'agir de séquences promotrices issues du génome de la cellule que l'on désire transfecter. Parmi les promoteurs eucaryotes, on peut utiliser tout promoteur ou séquence dérivée stimulant ou réprimant la transcription d'un gène de façon spécifique ou non, forte ou faible. Il peut s'agir en particulier de promoteurs ubiquitaires (HPRT, vimentine, α-actine, tubuline, etc.) , de promoteurs de gènes thérapeutiques (type MDR, CFTR, etc.), de promoteurs spécifiques de tissus (type promoteurs des gènes de la desmine, des myosines, de créatine kinase, de phophoglycérate kinase) ou encore de promoteurs répondant à un stimulus tels que des promoteurs répondant aux hormones naturelles (récepteur des hormones stéroïdes, récepteur de l'acide rétinoïque, etc.) ou un promoteur régulé par les antibiotiques (tétracycline, rapamycine, etc ), de promoteurs répondant à un régime alimentaire comme les promoteurs répondant aux fibrates, ou d'autres promoteurs répondant à d'autres molécules d'origine naturelle ou synthétique. De même, il peut s'agir de séquences promotrices issues du génome d'un virus. A cet égard, on peut citer par exemple les promoteurs des gènes EIA de l'adénovirus, MLP, ou de promoteurs issus des génomes des virus CMV, RSV, SV40, etc. Il peut s'agir de promoteurs inductibles ou répressibles. En outre, ces séquences d'expression peuvent être modifiées par addition de séquences d'activation, de régulation, permettant une expression conditionnelle, transitoire, une expression tissu-spécifique ou majoritaire, etc.

Par ailleurs, l'acide nucléique peut également comporter, en particulier en amont du gène thérapeutique, une séquence signal dirigeant le produit thérapeutique synthétisé dans les voies de sécrétion de la cellule cible. Cette séquence signal peut être la séquence signal naturelle du produit thérapeutique, mais il peut également s'agir de toute autre séquence signal fonctionnelle, ou d'une séquence signal artificielle. L'acide nucléique peut également comporter une séquence signal dirigeant le produit thérapeutique synthétisé vers un compartiment particulier de la cellule, comme par exemple les peroxisomes, les lysosomes, et les mitochondries pour le traitement par exemple des maladies génétiques mitochondriales.

D'autres gènes présentant un intérêt ont été notamment décrits par McKusick, V.A. Mendelian (Inheritance in man, catalogs of autosomal dominant, autosomal recessive, and X-linked phenotypes. Eighth edition. John Hopkins University Press (1988)), et dans Stanbury, J.B. et al. (The metabolic basis of inherited disease, Fith edition. McGraw-Hill (1983)). Les gènes d'intérêt recouvrent les protéines impliquées dans le métabolisme des acides aminés, des lipides et des autres constituants de la cellule.

On peut ainsi citer de manière non limitative les gènes associés aux maladies du métabolisme des carbohydrates comme par exemple fructose-1-phosphate aldolase, fructose-1,6-diphosphatase, glucose-6-phosphatase, α-1,4-glucosidase lysosomale, amylo-1,6-glucosidase, amylo-(1,4 :1,6)-transglucosidase, phosphorylase musculaire, phosphofructokinase musculaire, phosphorylase-b-kinase, galactose-1-phosphate uridyl transférase, toutes les enzymes du complexe pyruvate déshydrogénase, pyruvate carboxylase, 2-oxoglutarate glyoxylase carboxylase, D-glycérate déhydrogénase.

On peut également citer:
- les gènes associés avec des maladies du métabolisme des amino-acides comme par exemple phénylalanine hydroxylase, dihydrobioptérine synthétase, tyrosine aminotransférase, tyrosinase, histidinase, fumarylacéto-acétase, glutathion synthétase, γ-glutamylcystéine synthétase, ornithine-δ-aminotransférase, carbamoylphosphate synthétase, ornithine carbamoyltransférase, argininosuccinate synthétase, argininosuccinate lyase, arginase, L-lysine déhydrogénase, L-lysine kétoglutarate réductase, valine transaminase, leucine isoleucine transaminase, décarboxylase des 2-céto-acides à chaîne ramifiée, isovaléryl-CoA déhydrogénase, acyl-CoA déhydrogénase, 3-hydroxy-3-méthylglutaryl-CoA lyase, acétoacétyl-CoA 3-kétothiolase, propionyl-CoA carboxylase, méthylmalonyl-CoA mutase, ATP :cobalamine adénosyltransférase, dihydrofolate réductase, méthylène tétrahydrofolate réductase, cystathionine β-synthétase, le complexe sarcosine déshydrogénase, les protéines appartenant au système de clivage de la glycine, β-alanine transaminase, camosinase sérique, homocarnosinase cérébrale.
- Les gènes associés avec des maladies du métabolisme des graisses et des acides gras, comme par exemple lipoprotéine lipase, apolipoprotéine C-II, apolipoprotéine E, d'autres apolipoprotéines, lécithine cholestérolacyltransférase, récepteur des LDL, stérol hydroxylase du foie, « acide phytanique » α-hydroxylase.
- Les gènes associés avec des déficiences lysosomales, comme par exemple α-L-iduronidase lysosomale, iduronate sulfatase lysosomale, héparan N-sulfatase lysosomale, N-acétayl-α-D-glucosaminidase lysosomale, acétyl-CoA: α-glucosamine N-acétyltransférase lysosomale, N-acétyl-α-D-glucosamine 6-sulfatase lysosomale, galactosamine 6-sulfate sulfatase lysosomale, β-galactosidase lysosomale, arylsulfatase B lysosomale, β-glucuronidase lysosomale, N-acétylglucosaminyl-phosphotransférase, α-D-mannosidase lysosomale, α-neuraminidase lysosomale, aspartylglycosaminidase lysosomale, α-L-fucosidase lysosomale, lipase acide lysosomale, céramidase acide lysosomale, sphingomyelinase lysosomale, glucocérébrosidase lysosomale et galactocérébrosidase lysosomale, galactosylcéramidase lysosomale, arylsulfatase A lysosomale, α-galactosidase A, β-galactosidase acide lysosomale, chaîne α de l'hexosaminidase A lysosomale.

On peut également citer, de façon non restrictive, les gènes associés avec des maladies du métabolisme des stéroïdes et des lipides, les gènes associés avec des maladies du métabolisme des purines et des pyrimidines, les gènes associés à des maladies du métabolisme de la porphyrine et de l'hème, les gènes associés à des maladies du métabolisme du tissu conjonctif, des s et des os ainsi que les gènes associés avec des maladies du sang et des organes hématopoïétiques, des muscles (myopathie), du système nerveux (maladies neurodégénératives) ou de l'appareil circulatoire (traitement des ischémies et de la sténose par exemple) et les gènes impliqués dans les maladies génétiques mitochondriales.

Dans le procédé suivant l'invention, l'acide nucléique peut être associé à tout type de vecteurs ou toute combinaison de ces vecteurs permettant d'améliorer le transfert de gènes, par exemple, de façon non limitative, à des vecteurs tels que des virus, des agents synthétiques ou biosynthétiques (par exemple lipidiques, polypeptidiques, glycosidiques ou polymériques), ou encore des billes propulsées ou non. Les acides nucléiques peuvent aussi être injectés dans un tissu qui a été soumis à un traitement visant à améliorer le transfert de gènes, par exemple un traitement de nature pharmacologique en application locale ou systémique, ou un traitement enzymatique, perméabilisant (utilisation de tensioactifs), chirurgical, mécanique, thermique ou physique.

L' avantage de l' utilisation de l'éléctrotransfert en thérapie génique réside dans la sécurité apportée par le traitement local lié à l'utilisation de champs électriques locaux et ciblés.

De par la sécurité liée à l'utilisation de champs faibles, la présente invention pourrait s'appliquer au niveau du muscle cardiaque pour le traitement de cardiopathies, par exemple en utilisant un défibrilateur adapté. Elle pourrait s'appliquer aussi au traitement de la resténose par l'expression de gènes inhibiteurs de la prolifération des cellules musculaires lisses comme la protéine GAX.

La combinaison de champs peu intenses et de durées d'administration longues appliquée aux tissus in vivo améliore la transfection des acides nucléiques sans amener de détériorations notables des tissus. Ces résultats améliorent le rendement des transferts d'ADN dans le cadre de la thérapie génique mettant en oeuvre les acides nucléiques.

En conséquence, le procédé selon l'invention permet, pour la première fois, d'envisager de produire par thérapie génique un agent à des doses physiologiques et/ou thérapeutiques, soit dans les tissus, soit sécrété dans leur voisinage ou dans la circulation sanguine ou lymphatique. De plus, le procédé selon l'invention permet, pour la première fois, la modulation fine et le contrôle de la quantité efficace de transgène exprimé par la possibilité de moduler le volume du tissu à transfecter, par exemple avec des sites multiples d'administration, ou encore la possibilité de moduler le nombre, la forme, la surface et la disposition des électrodes. Un élément de contrôle supplémentaire provient de la possibilité de moduler l'efficacité de la transfection par la variation de l'intensité de champ, du nombre de la durée et de la fréquence des impulsions, et évidemment suivant l'état de l'art, la quantité et le volume d'administration des acides nucléiques. On peut ainsi obtenir un niveau de transfection approprié au niveau de production ou de sécrétion désiré. Le procédé permet enfin un surcroît de sécurité par rapport aux méthodes chimiques ou virales de transfert de gènes in vivo, pour lesquelles l'atteinte d'organes autres que l'organe cible ne peut pas être totalement exclue et maîtrisée. En effet, le procédé selon l'invention permet le contrôle de la localisation des tissus transfectés (strictement liée au volume de tissu soumis aux impulsions électriques locales) et apporte donc la possibilité d'un retour à la situation initale par l'ablation totale ou partielle du tissu lorsque cela est rendu possible par le caractère non vital de ce tissu et par ses capacités de régénération comme dans le cas du foie ou du muscle. Cette grande souplesse d'utilisation permet d'optimiser le procédé suivant l'espèce animale (applications humaines et vétérinaires), l'âge du sujet, son état physiologique et/ou pathologique.

Le procédé selon l'invention permet, en outre, pour la première fois, de transfecter des acides nucléiques de grande taille contrairement aux méthodes virales qui sont limitées par la taille de la capside. Cette possibilité est essentielle pour le transfert de gènes de très grande taille comme celui de la dystrophine ou de gènes avec des introns et/ou des éléments régulateurs de grande taille, ce qui est nécessaire par exemple pour une production physiologiquement régulée d'hormones. Cette possibilité est essentielle pour le transfert d'épisomes ou de chromosomes artificiels de levure ou de minichromosomes.

Les exemples qui suivent sont destinés à illustrer de manière non limitative l'invention.

Dans ces exemples, on se réfèrera aux figures suivantes :
- Figure 1 : Effets d'impulsions électriques d'intensité de champ élevé sur la transfection d'ADN plasmidique pXL2774 dans le muscle tibial cranial chez la souris; valeurs moyennes ± SEM,
- Figure 2 : Effets d'impulsions électriques d'intensité de champ intermédiaire sur la transfection d'ADN plasmidique pXL2774 dans le muscle tibial cranial chez la souris; valeurs moyennes ± SEM,
- Figure 3 : Effets d'impulsions électriques d'intensité de champ faible et de différentes durées sur la transfection d'ADN plasmidique pXL2774 dans le muscle tibial cranial chez la souris; valeurs moyennes ± SEM,
- Figure 4 : Effets d'impulsions électriques d'intensité de champ faible et de différentes durées sur la transfection d'ADN plasmidique pXL2774 dans le muscle tibial cranial chez la souris; valeurs moyennes ± SEM,
- Figure 5 : Efficacité de l'électrotransfection de l'ADN plasmidique pXL2774 dans le muscle tibial cranial de la souris aux intensités de champs électriques faibles: valeurs moyennes ± SEM.
- Figure 6 : Carte des plasmides pXL3031 et pXL3010.

### Exemple 1 : Expérience effectuée dans les conditions de l'état de la technique antérieure dans laquelle les champs électriques se montrent inhibiteurs de la transfection

Les conditions standards d'électroporation, telles que celles utilisées dans l'art antérieur et qui ont été discutées ci-avant, ont été testées et se sont avérées être inefficaces, voire même avoir une action inhibitrice sur le transert d'acides nucléiques (ADN plasmidique) dans le muscle strié.

### Matériel et Méthodes - Conditions opératoires générales

Dans cet exemple, les produits suivants ont été utilisés :

ADN pXL2774 (brevet PCT/FR 96/01414) est un ADN plasmidique comportant le gène rapporteur de la luciférase. Les autres produits sont disponibles auprès de fournisseurs du commerce : Kétamine, Xylazine, Sérum physiologique (NaCl 0,9 %).

Un oscilloscope et un générateur d'impulsions électriques (rectangulaires ou carrées) du commerce (Electro-pulsateur PS 15, Jouan, France) ont été utilisés. Les électrodes utilisées sont des électrodes plates en acier inoxydable distantes de 5,3 mm.

L'expérience est réalisée chez la souris C57 B1/6. Les souris provenant de différentes cages sont réparties au hasard avant l'expérience ("randomisation").

Les souris sont anesthésiées par un mélange kétamine, xylazine. La solution de plasmide (30 µl d'une solution à 500 µg/ml de NaCl 0,9%) est injectée longitudinalement à travers la peau dans le muscle tibial cranial des pattes gauche et droite à l'aide d'une seringue hamilton. Les deux électrodes sont enduites d'un gel conducteur et la patte injectée est placée entre les électrodes au contact de celles-ci.

Les impulsions, électriques sont appliquées perpendiculairement à l'axe du muscle à l'aide d'un générateur d'impulsions carrées, une minute après l'injection. Un oscilloscope permet de contrôler l'intensité en Volts (les valeurs indiquées dans les exemples représentent les valeurs maximales), la durée en millisecondes et la fréquence en hertz des impulsions délivrées, qui est de 1 Hz. 8 impulsions consécutives sont délivrées.

Pour l'évaluation de la transfection du muscle, les souris sont euthanasiées 7 jours après l'administration du plasmide. Les muscles tibial cranial des pattes gauche et droite sont alors prélevés, pesés, mis dans du tampon de lyse et broyés. La suspension obtenue est centrifugée afin d'obtenir un surnageant clair. La mesure de l'activité luciférase est réalisée sur 10 µl de surnageant à l'aide d'un luminomètre du commerce dans lequel le substrat est ajouté automatiquement à la solution. L'intensité de la réaction lumineuse est donnée en RLU (Relative Luminescence Unit) pour un muscle connaissant le volume total de suspension. Chaque condition expérimentale est testée sur 10 points : 5 animaux injectés en bilatéral. Les comparaisons statistiques sont réalisées à l'aide de tests non paramétriques.

### Résultats et discussion.

Deux figures, dont l'échelle est linéaire ou logarithmique, illustrent les résultats.

Dans cette première expérience on a testé les effets d'un champ électrique de 800 à 1200 Volts/cm qui permet l'électroporation de tumeurs (Mir et al. Eur. J. Cancer 27, 68,1991).

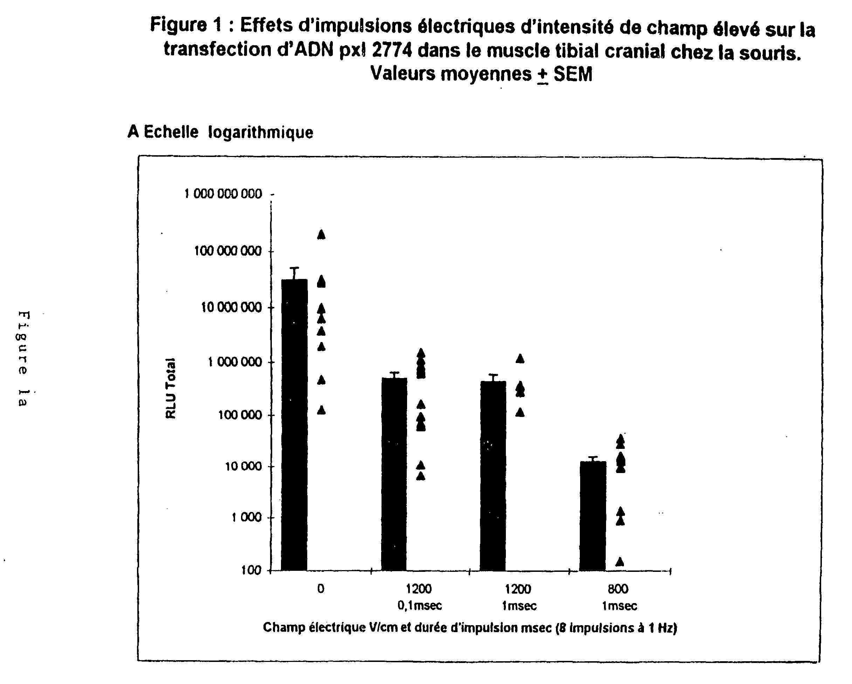

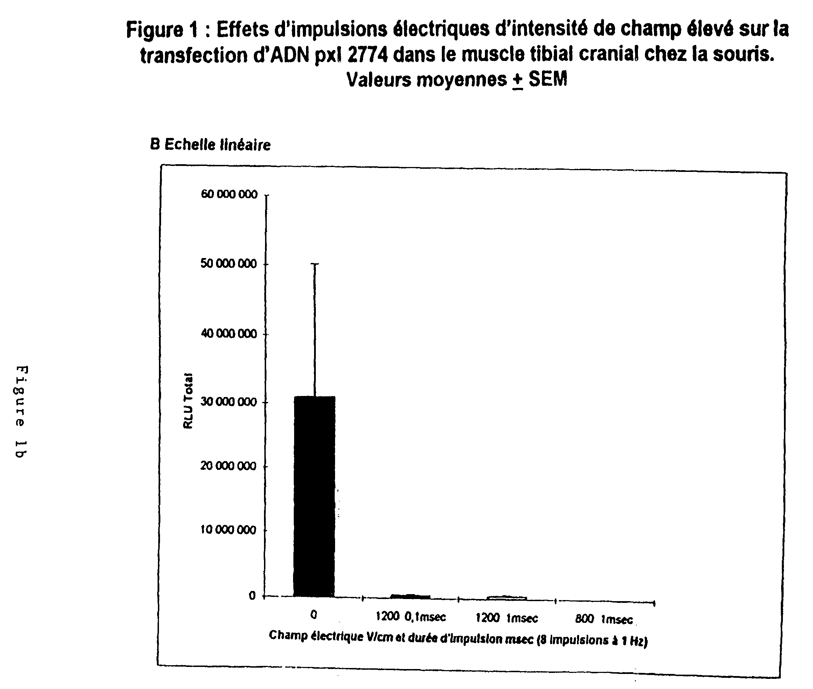

On constate, d'après la figure 1, que, relativement au groupe contrôle, où l'ADN est injecté sans impulsion électrique :
- avec 8 impulsions de 1200 Volts/cm et d'une durée de 0,1 msec, la valeur moyenne de l'activité luciférase est beaucoup plus faible,
- avec des impulsions de 1200 Volts/cm et de 1 msec, 3 animaux sont morts, la valeur moyenne de l'activité luciférase est beaucoup plus faible,
- avec des impulsions de 800 Volts/cm et de 1 msec la valeur moyenne de l'activité luciférase est aussi significativement réduite.

La plupart des muscles ayant subi l'action du champ électrique sont visiblement altérés (friables et d'aspect blanchâtre).

### Exemple 2 de référence : expérience d'électrotransfert d'acides nucléiques à des champs électriques modérés

Cette expérience est réalisée avec des souris C57 B1/6. Mis à part l'intensité de champ électrique des impulsions et leur durée, les conditions de réalisation sont celles de l'exemple 1.

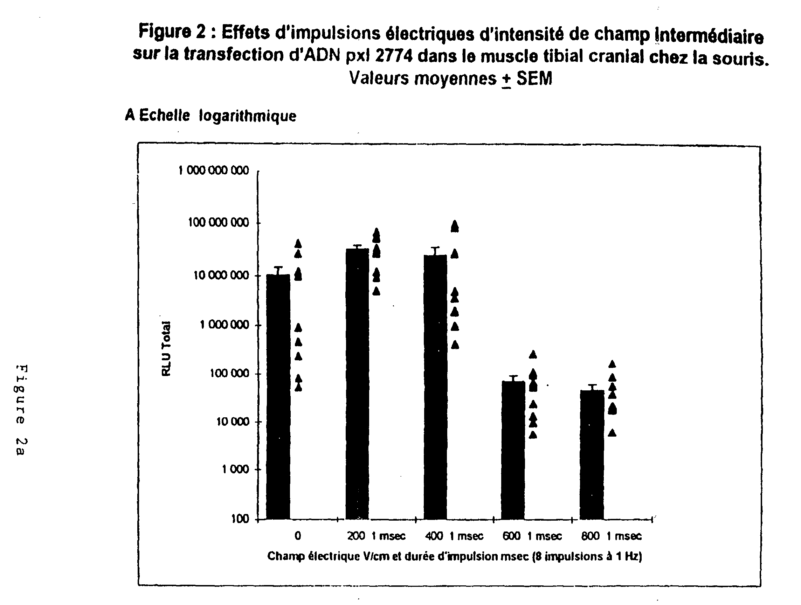

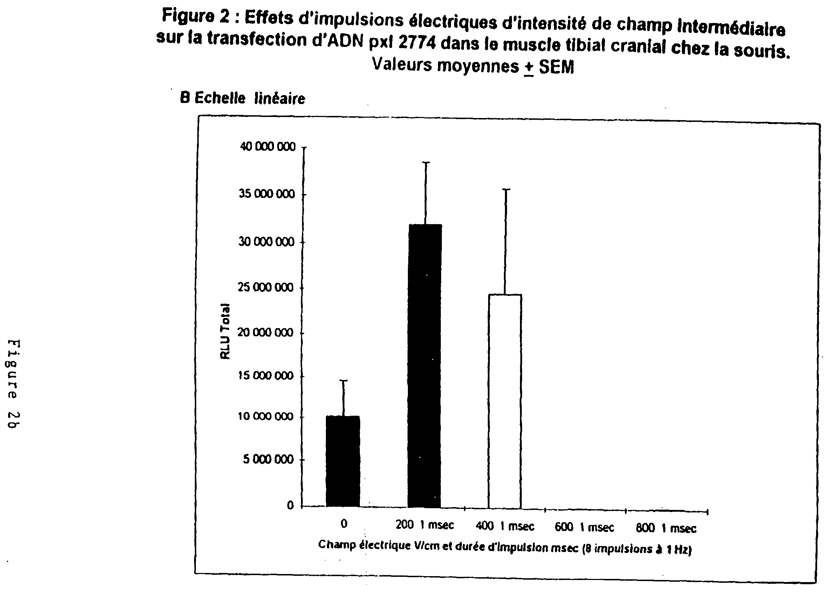

Les résultats sont montrés à la figure 2. On reproduit le résultat de l'exemple 1, c'est-à-dire l'effet inhibiteur d'une série de 8 impulsions à 800 Volts/cm d'une durée de 1 msec sur l'activité luciférase détectée dans le muscle. Avec un champ de 600 Volts/cm, on observe la même inhibition et la même altération du tissu musculaire. Par contre, de façon remarquable et surprenante, la diminution du voltage permet de ne plus altérer visiblement les muscles, et, de plus, à 400 et 200 Volts/cm le niveau de transfection des muscles est en moyenne supérieur à celui obtenu sur les muscles non soumis à un champ. Il est à noter que, relativement au groupe témoin (non soumis à un champ électrique), la dispersion des valeurs de l'activité luciférase est diminuée à 200 Volts/cm (SEM = 20,59% de la valeur moyenne contre 43,32% en l'absence de champ électrique (figure 2A)).

### Exemple 3 de référence : expérience d'électrotransfert d'acides nucléiques avec des impulsions de faible intensité de champ montrant une très forte stimulation de l'expression du transgène

Cette expérience est réalisée avec des souris C57 B1/6. Mis à part l'intensité de champ électrique des impulsions et leur durée, et le fait que les impulsions sont délivrées 25 secondes après l'injection de l'ADN, les conditions de réalisation sont celles des exemples précédents.

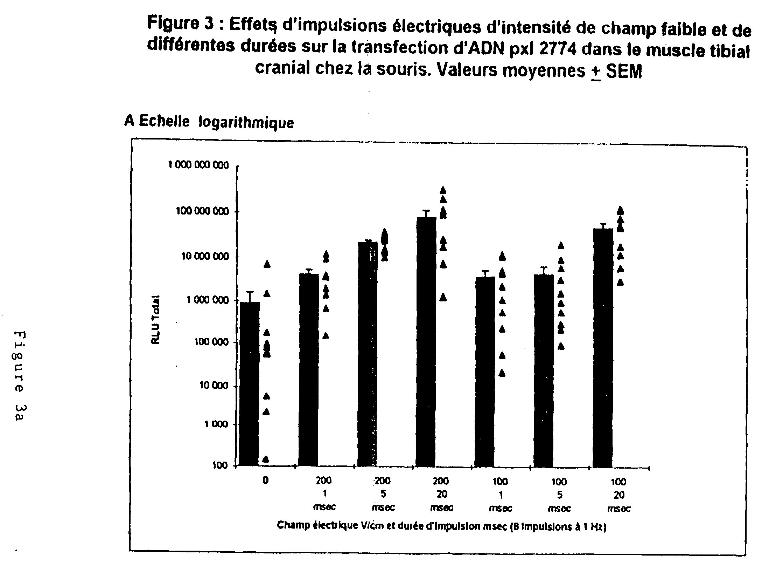

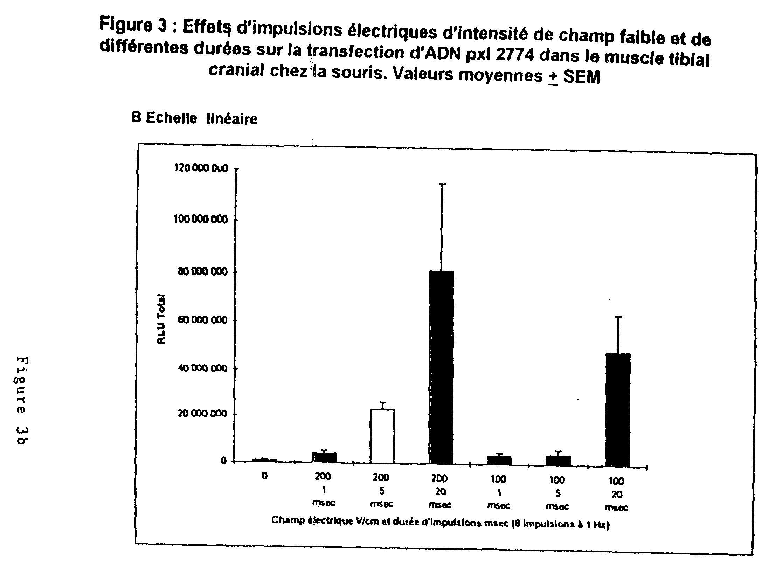

Les résultats sont montrés à la figure 3. La valeur moyenne de l'expression du transgène luciférase est nettement augmentée avec une durée d'impulsion de 20 msec à 100 Volts/cm, et à partir d'une durée d'impulsion de 5 msec à 200 Volts/cm.

Cette expérience montre aussi clairement que la valeur moyenne de l'activité luciférase obtenue par électrotransfection de l'ADN dans le muscle est une fonction de la durée des impulsions électriques, lorsqu'on emploie des voltages de 200 et 100 Volts/cm. On note aussi que la dispersion des valeurs est notablement réduite pour les groupes de muscles électrotransfectés (figure 3A). En l'absence d'impulsions électriques (contrôle), la SEM représente 77,43% de la valeur moyenne alors que la SEM relative de la moyenne est réduite à 14% (200 Volts/cm, 5 msec), 41,27% (200 Volts/cm, 20 msec) et entre 30% et 48% pour l'électrotransfert à 100 Volts/cm de champ électrique.

Dans la meilleure condition de cette expérience, on améliore par un facteur de 89,7 l'expression du transgène par rapport au contrôle injecté en l'absence d'impulsions électriques.

### Exemple 4 de référence : expérience d'électrotransfert d'acides nucléiques dans le muscle à 200 Volts/cm montrant une augmentation de l'expression du transgène d'un facteur supérieur à 200

Cette expérience est effectuée chez les souris DBA 2, avec des impulsions électriques d'une intensité de champ de 200 Volts/cm et de durée variable, les autres conditions de cette expérience étant celles de l'exemple 3.

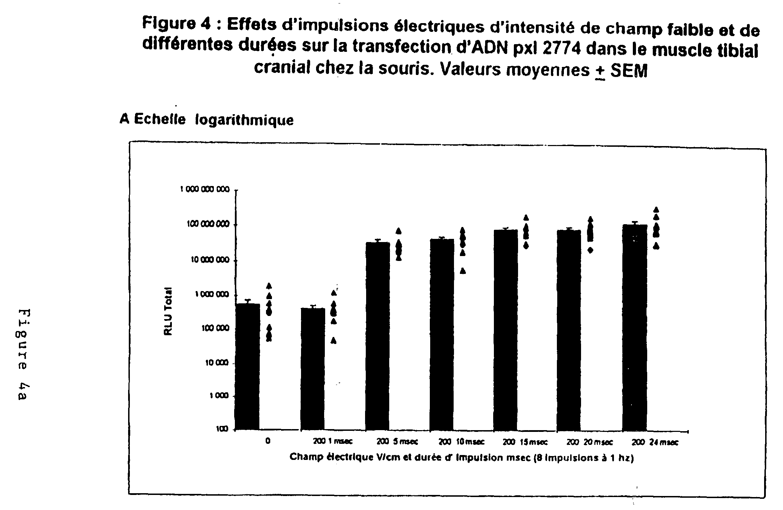

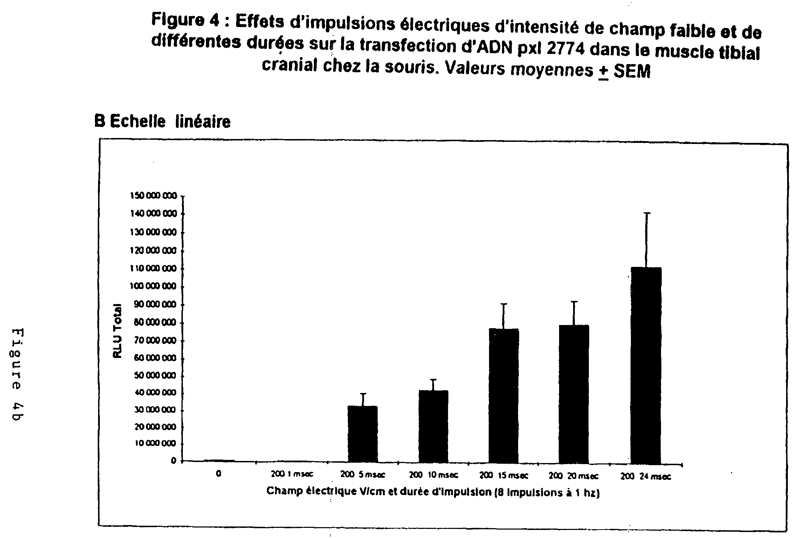

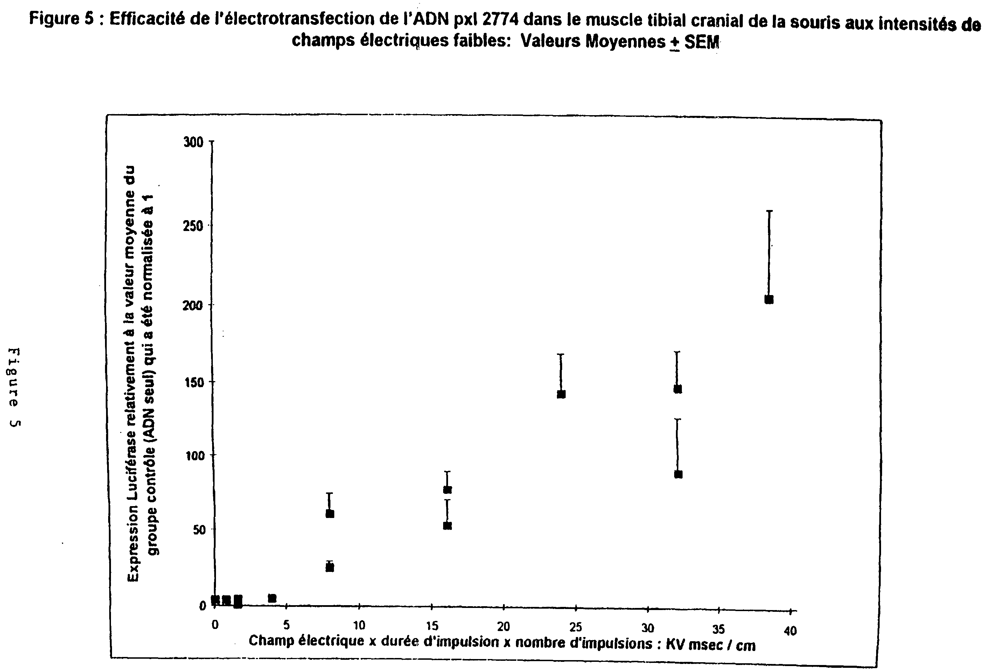

Cet exemple confirme qu'à 200 Volts/cm la transfection de l'activité luciférase est augmentée à partir d'une durée d'impulsion de 5 msec puis continue à croître pour des durées plus longues (figures 4 et 5). Là encore, on observe avec l'électrotransfection une réduction de la variabilité inter-individuelle indiquée par la SEM par rapport au contrôle non électrotransfecté (la valeur relative de la SEM est égale à 35% pour le contrôle et 25, 22,16,18, 16 et 26% pour des séries d'impulsions de 1, 5, 10, 15, 20, et 24 msec respectivement).

Dans la meilleure condition de cette expérience, on améliore par un facteur de 205 l'expression du transgène par rapport au contrôle injecté en l'absence d'impulsions électriques.

### Exemple 5 de référence : efficacité de l'électrotransfert d'acides nucléiques en fonction du produit « nombre des impulsions x intensité du champ x durée de chaque impulsion »

La figure 5 exemplifie l'importance du paramètre correspondant au produit « nombre des impulsions x intensité du champ x durée de chaque impulsion ». Ce paramètre correspond en fait à l'intégrale en fonction du temps de la fonction qui décrit la variation du champ électrique.

La représentation en figure 5 des résultats obtenus au cours des expériences 2, 3 et 4 avec des intensités de champ électrique de 200 V/cm, 100 V/cm ou en absence des champs électriques montre que l'efficacité de transfection augmente en fonction du produit de la durée totale d'exposition au champ électrique par l'intensité de champ. Un effet de stimulation est obtenu pour une valeur supérieure à 1 kV xmsec/cm du produit « champ électrique x durée totale des impulsions ». Selon un mode préféré, une stimulation est obtenue pour une valeur supérieure ou égale à 5 kV xmsec/cm du produit « champ électrique x durée totale des impulsions ».
Dans les exemples qui suivent, l'électrotransfert d'acides nucléiques au moyen du procédé selon l'invention a été testé sur différentes tumeurs soit d'origine humaine implantée sur des souris nude (immunodéficientes) soit d'origine murine implantées sur des souris C57B1/6 (immunocompétentes).

### Exemple 6 : expérience d'électrotransfert d'acides nucléiques dans des tumeurs pulmonaires humaines H1299

L'expérience est réalisée chez la souris nude femelle de 18 à 20g. Les souris sont implantées monolatéralement avec des greffons de tumeurs H1299 de 20mm³. Les tumeurs se développent pour atteindre un volume de 200 à 300mm³. Les souris sont triées en fonction de leurs tailles tumorales et réparties en lots homogènes. Les souris sont anesthésiées avec un mélange Kétamine, Xylazine. La solution de plasmide (40µl d'une solution à 250µg/ml ADN dans NaCl 20mM, 5% glucose ) est injectée longitudinalement au centre de la tumeur à l'aide d'une seringue Hamilton. Les faces latérales de la tumeur sont enduites de gel conducteur et la tumeur est placée entre les deux électrodes. Les électrodes sont des électrodes plates en acier inoxidable distantes de 0.45 à 0.7 cm. Un oscilloscope et un générateur d'impulsions électriques (rectangulaires ou carrées) du commerce (Electro-pulsateur PS 15, Jouan, France) ont été utilisés.

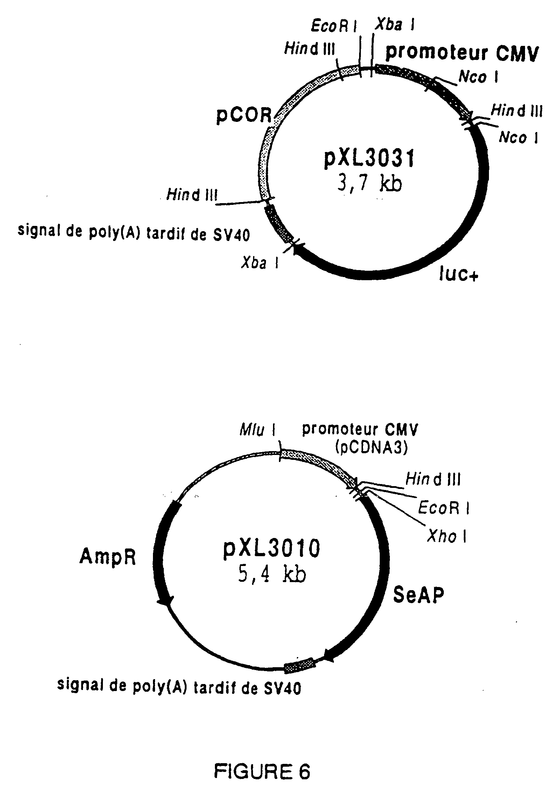

Dans cet exemple le plasmide utilisé est le plasmide pXL3031 (Figure 6) comportant le gène codant pour la luciférase (cytoplasmique). Le plasmide pXL3031 est un vecteur dérivé du vecteur pXL2774 (WO97/10343) dans lequel le gène luc+ codant pour la luciférase de Photinus pyralis modifiée (cytoplasmique) provenant de pGL3basic (Genbank: CVU47295) a été introduit sous contrôle du promoteur provenant de la région précoce du cytomegalovirus humain (hCMV IE, Genbank HSSIEE) et du signal de poly-adénylation de la région tardive du virus SV40 (Genbank SV4CG).

Les impulsions électriques sont appliquées à l'aide d'un générateur d'impulsions carrées, 20 à 30 sec après l'injection. Un oscilloscope permet de contrôler l'intensité en Volts, la durée en millisecondes et la fréquence en hertz des impulsions délivrées soit 200 à 800 Volts/cm, 20 msec et 1 hertz.

Pour l'évaluation de la transfection tumorale, les souris (10 souris par conditions) sont euthanasiées 2 jours après l'injection du plasmide. Les tumeurs sont prélevées pesées et broyées dans un tampon de lyse. La suspension obtenue est centrifugée afin d'obtenir un surnageant clair. L'activité luciférase est mesurée dans 10 µl de surnageant à l'aide d'un luminomètre du commerce dans lequel le substrat est ajouté automatiquement. Les résultats sont exprimés en RLU (Relative light Unit) totaux par tumeurs.

Dans cet exemple, deux séries d'expériences ont été réalisées pour déterminer l'effet de l'intensité du champ électrique sur l'efficacité de la transfection dansles tumeurs pulmonaires humaines H1299. Dans une première série d'expériences, des intensités de champ électrique de 200 à 500 Volts/cm ont été testées. Dans une deuxième série d'expériences, des intensités de champ électrique variant de 400 à 800 Volts / cm ont été testées.

**Tableau 1 :**

| Effet d'impulsions électriques de différentes intensités de champs sur la transfection d'ADN plasmidique pXL 3031 sur des tumeurs humaines H1299 (carcinomes pulmonaires non à petites cellules); valeurs moyennes +/- SEM de l'activité luciférase en RLU par tumeur. Conditions : intensité de champ électrique V/cm comme indiquée dans le tableau, 8 impulsions de 20 msec, fréquence 1 Hertz. | | | | |
|---|---|---|---|---|
| | **Expérience 1** | | **Expérience 2** | |
| **Volt/cm** | **Moyenne** | **SEM** | **Moyenne** | **SEM** |
| 0 | 32,8 | ± 6,8 | 44,7 | ± 10,2 |
| 200 | 129,7 | ± 39,1 | | |
| 300 | 585,0 | ± 134,8 | | |
| 400 | 5 266,6 | ± 1473,8 | 8488,2 | ±3881,7 |
| 500 | | | 14201,6 | ± 6 162,6 |
| 600 | | | 7401,0 | ± 5 323,1 |
| 800 | | | 11 884,1 | ± 4 048,3 |

On constate , d'après le tableau 1, que relativement au groupe contrôle où l'ADN est injecté sans impulsion électrique, le transfert de gène est augmenté d'une manière dépendante de l'intensité du champ électrique de 200 à 400 Volts/cm pour atteindre un plateau correspondant au maximum de transfection obtenu dès 500 volts/cm. A des voltages plus élevés (600 et 800 volts/cm), des brûlures cutanées ou plus profondes sont obtenues sans toutefois diminuer l'expression du transgène.

L'amplification du transfert de gène obtenue par électrotransfert dans les tumeurs pulmonaires H1299 est de l'ordre de 240 à 320 fois.

### Exemple 7 : expérience d'électrotransfert d'acides nucléiques dans des tumeurs humaines de colon HT29

L'expérience est réalisée chez la souris nude femelle de 18 à 20g. Les souris sont implantées monolatéralement avec des greffons de tumeurs HT29 de 20mm³. Les tumeurs se développent pour atteindre un volume de 100 à 200mm³. Les souris sont triées en fonction de leurs tailles tumorales et réparties en lots homogènes. Mis à part l'écartement des électrodes utilisé (0.45 cm), les conditions de réalisations sont celles de l'exemple 6. Les résultats de deux séries d'expériences indépendantes sont présentés dans le tableau 2.

**Tableau 2 :**

| Effet d'impulsions électriques de différentes intensités de champ sur la transfection d'ADN plasmidique pXL 3031 sur des tumeurs humaines HT29 (adénocarcinomes de colon); valeurs moyennes +/- SEM de l'activité luciférase en RLU par tumeur. Conditions : intensité de champ électrique V/cm comme indiquée dans le tableau, 8 impulsions de 20 msec, fréquence 1 Hertz. | | | | |
|---|---|---|---|---|
| | **Expérience 1** | | **Expérience 2** | |
| **Volt/cm** | **Moyenne** | **SEM** | **Moyenne** | **SEM** |
| 0 | 4,0 | ± 1,8 | 0,6 | ± 0,3 |
| 400 | 16,0 | ± 5,4 | | |
| 500 | 14,1 | ± 7,6 | 5,5 | ± 3,6 |
| 600 | 24,2 | + 9,2 | 14,6 | ± 6,4 |

Comparativement aux groupes contrôles sans électrotransfert, l'application d'un champ électrique d'une intensité de 600 volts/cm permet d'atteindre un taux de transfection optimal quelque soit le niveau de base de transfection sans électrotransfert. L'amélioration de la transfection est d'un facteur 6 à 23 fois respectivement, et est relativement similaire de 400 à 600 Volts/cm.

### Exemple 8 : expérience d'électrotransfert d'acides nucléiques dans des fibrosarcomes murins

L'expérience est réalisée chez la souris C57B1/6 de 18 à 20g. Les souris sont implantées monolatéralement avec 1 x 10⁶ cellules LPB dans 100µl de milieu MEM sans sérum. Les tumeurs se dévelopent pour atteindre un volume de 100 à 200mm³. Les souris sont triées en fonction de leurs tailles tumorales et réparties en lots homogènes.Les conditions de réalisations de l'expérience sont celles de l'exemple 6.

Les résultats de deux séries d'expériences indépendantes sont présentés dans le tableau 3.

**Tableau 3 :**

| Effet d'impulsions électriques de différentes intensités de champ sur la transfection d'ADN plasmidique pXL 3031 sur des fibrosarcomes murins; valeurs moyennes +/- SEM de l'activité luciférase en RLU par tumeur. Conditions : intensité de champ électrique V/cm comme indiquée dans le tableau, 8 impulsions de 20 msec, fréquence 1 Hertz. | | | | |
|---|---|---|---|---|
| | **Expérience 1** | | **Expérience 2** | |
| **Volt/cm** | **Moyenne** | **SEM** | **Moyenne** | **SEM** |
| 0 | 0,6 | ± 0,3 | 0,4 | ± 0,1 |
| 300 | 26,3 | ± 14,8 | 11,6 | ± 4,6 |
| 400 | 42,5 | ± 31,2 | 10,4 | ± 3,5 |
| 500 | 17,0 | ± 12,8 | 6,0 | ± 1,8 |
| 600 | | | 11,0 | ± 7,1 |

Comparativement aux groupes contrôles sans électrotransfert, l'application d'un champ électrique d'une intensité de 300 à 600 Volts/cm permet d'améliorer le transfert de gène d'un facteur 30 à 70 , quelque soit le voltage appliqué.

### Exemple 9 : expérience d'électrotransfert d'acides nucléiques dans des mélanomes B16 murins

L'expérience est réalisée chez la souris C57B1/6 de 18 à 20g. Les souris sont implantées monolatéralement avec des greffons de tumeurs B16 de 20mm³. Les tumeurs se développent pour atteindre un volume de 200 à 300mm³. Les souris sont triées en fonction de leurs tailles tumorales et réparties en lots homogènes.

Les conditions de réalisations de l'expérience sont celles de l'exemple 6.

Les résultats sont présentés dans le tableau 4.

**Tableau 4 :**

| Effet d'impulsions électriques de différentes intensités de champ sur la transfection d'ADN plasmidique pXL 3031 sur des mélanomes murins B16; valeurs moyennes +/- SEM de l'activité luciférase en RLU par tumeur. Conditions : intensité de champ électrique V/cm comme indiquée dans le tableau, 8 impulsions de 20 msec, fréquence 1 Hertz. | | |
|---|---|---|
| | **Expérience 1** | |
| **Volt/cm** | **Moyenne** | **SEM** |
| 0 | 1,3 | ± 0,7 |
| 300 | 14,3 | ± 7,6 |
| 500 | 32,2 | ± 12,6 |
| 600 | 17,2 | ± 6,2 |

Comparativement au groupe contrôle sans électrotransfert, l'application d'un champ électrique d'une intensité de 500 Volts/cm permet d'améliorer le transfert de gène d'un facteur 24.

### Exemple 10 : expérience d'électrotransfert d'acides nucléiques dans des tumeurs 3LL murines

L'expérience est réalisée chez la souris C57B1/6 de 18 à 20g. Les souris sont implantées monolatéralement avec des greffons de tumeurs 3LL de 20mm³.

La taille des tumeurs transfectées obtenues cinq jours après l'implantation est de 30 mm³. Les conditions de réalisations de l'expérience sont celles de l'exemple 6. Les résultats sont présentés dans le tableau 5.

**Tableau 5 :**

| Effet d'impulsions électriques de différentes intensités de champ sur la transfection d'ADN plasmidique pXL 3031 sur des carcinomes pulmonaires murins 3LL; valeurs moyennes +/- SEM de l'activité luciférase en RLU par tumeur. Conditions : intensité de champ électrique V/cm comme indiquée dans le tableau, 8 impulsions de 20 msec, fréquence 1 Hertz. | | |
|---|---|---|
| **Volt/cm** | **Moyenne** | **SEM** |
| 0 | 0,1 | ± 0,04 |
| 300 | 3,7 | ± 2,9 |
| 500 | 470,5 | ± 237,6 |
| 600 | 53,3 | ± 23,9 |

L'application d'un champ électrique d'une intensité de 500 Volts/cm permet d'augmenter l'expression du transgène d'un facteur 3885.

Ces résultats remarquables sont à mettre en relation avec le fait que ces tumeurs sont très peu transfectables par l'ADN lorsque l'ADN est simplement injecté sans électrotransfert.

### Exemple 11 : expérience d'électrotransfert d'acides nucléiques dans des tumeurs pulmonaires humaines H1299, effet sur la sécrétion dans le plasma de la phosphatase alkaline humaine sécrétée.

Dans cet exemple, l'ADN pXL3010 (figure 6) utilisé est un ADN plasmidique comportant le gène codant pour la phosphatase alkaline humaine placentaire sécrétée.

Le plasmide pXL3010 est un vecteur dérivé de ColE1 dans lequel le gène codant pour la phosphatase alcaline sécrétée provenant de pSEAP-basic (Clontech, Genbank: CVU09660) a été introduit sous contrôle du promoteur CMV provenant du plasmide pCDNA3 (Invitrogen, Pays-Bas) et du signal de poly-adénylation de la région tardive du virus SV40 (Genbank SV4CG).

L'expérience est réalisée chez la souris nude de 18 à 20g. Les souris sont implantées monolatéralement avec des greffons de tumeurs H1299 de 20mm³. Les tumeurs se développent pour atteindre un volume de 200 à 300mm³. Les souris sont triées en fonction de leurs tailles tumorales et réparties en lots homogènes.

Les tumeurs sont transfectées dans les conditions de réalisations de l'exemple 6 avec cependant une seule condition de voltage soit de 500 Volts/cm, 20 msec et 1 hertz.

Les dosages de la phosphatase alkaline sont réalisés dans le plasma à l'aide du kit Phospha-light (Tropix) au jour J1, J2 et J8 après la transfection avec ou sans électrotransfert. Les résultats sont présentés dans le tableau 6.

**Tableau 6 :**

| Effet d'impulsions électriques de différentes intensités de champ sur la sécrétion d'une protéine exogène: la phosphatase alkaline humaine sécrétée suite à la transfection d' ADN plasmidique pXL 3010 dans des tumeurs humaines H1299; valeurs moyennes +/- SEM de phosphatase alcaline (ng/ml). Conditions : intensité de champ électrique V/cm comme indiquée dans le tableau, 8 impulsions de 20 msec, fréquence 1 Hertz. | | |
|---|---|---|
| | Phosphatase alkaline dans le plasma | |
| **Prélèvement** | **0 Volt/cm (MOY+/- SEM)** | **500 Volts/cm (MOY+/- SEM)** |
| J1 | 1,42 ± 0,07 | 8,90 ± 1,74 |
| J2 | 1,40 ± 0,01 | 9,04 ± 1,55 |
| J8 | 1,31 ± 0,01 | 1,67 ± 1,12 |

L'ensemble des résultats présentés dans les exemple 6 à 11 montrent que l'électrotransfert d'acides nucléiques dans les conditions du procédé selon l'invention permet d'augmenter de façon remarquable le niveau d'expression du transgène, dans différents type de tumeurs. De plus , dans le cas d'un transgène codant pour une protéine sécrétée, l'administration intratumorale du plasmide par électrotransfert permet d'augmenter significativement la concentration plasmatique de la protéine sécrétée.

### Exemple 12 : effet de l'augmentation de la durée des impulsions électriques.

Cet exemple illustre que l'on peut augmenter la durée unitaire des impulsions bien au delà des valeurs testées dans l'exemple 4.

Cette expérience est réalisée avec des souris C57B1/6. Le plasmide utilisé est le plasmide pXL2774, la quantité d'ADN administrée est de 15 µg. L'électropulsateur utilisé pour délivrer les impulsions électrique d'une durée supérieure à 20 msec est un électropulsateur du commerce (Genetronics, modèle T 820, USA, San Diego, CA). Les impulsions électriques sont de nombre et de durée variable mais d'une intensité de champ constante de 200 Volts/cm ; les autres conditions de cette expérience sont celles décrites dans l'exemple 1. Les résultats sont présentés dans le tableau 7.

**Tableau 7 :**

| valeurs moyennes +/- SEM de l'activité luciférase en millions de RLU par muscle . N = 10 pour chaque groupe. Conditions électrotransfert : intensité de champ 200 V/cm, 8 ou 4 impulsions (durée unitaire variable), fréquence 1 Hz. | | | | | | | | | | |
|---|---|---|---|---|---|---|---|---|---|---|
| durée Impulsion (msec) | 0 | 1 | 5 | 10 | 20 | 30 | 40 | 50 | 60 | 80 |
| Expérience A 8 impulsions | 11 ± 5 | 39 ± 6 | 211 ± 26 | 288 ± 46 | 1158 ± 238 | 1487 ± 421 | 2386 ± 278 | | | |
| Expérience A 4 impulsions | 11 ± 5 | 26,8 ±6 | 123 ±17 | 246 ± 32 | 575 ± 88 | 704 ± 130 | | 3440 ±1077 | | |
| Expérience B 4 impulsions | 15 ±8 | | | | | | 2885 ±644 | | 2626 ±441 | 1258 ±309 |

On constate une augmentation de l'expression du transgène avec l'allongement de la durée unitaire des impulsions (au moins jusqu'à 40 msec pour une série de 8 impulsions et au moins jusqu'à 50 msec pour une série de 4 impulsions d'une intensité de 200 Volts/cm). Cet exemple montre que l'optimum de la durée des impulsions dépend du nombre d'impulsions utilisées et que la durée unitaire des impulsions peut atteindre au moins 80 msec, cette valeur de durée n'étant pas limitative.

### Exemple 13 : efficacité de l'électrotransfert en fonction du nombre d'impulsions électriques

Cette exemple met en évidence l'effet de l'augmentation du nombre d'impulsions électriques sur l'efficacité du transfert d'acides nucléiques.

Cette expérience est réalisée avec des souris C57B1/6. Le plasmide utilisé est le plasmide pXL 2774, la quantité d'ADN administrée est de 15 µg. Les impulsions électriques sont variables en nombre. La durée de chaque impulsion est de 20 msec. L'intensité de champ est de 200 Volts/cm. Les autres conditions de cette expérience sont celles décrites dans l'exemple 1. Les résultats sont présentés dans le tableau 8.

**Tableau 8 :**

| valeurs moyennes +/- SEM de l'activité luciférase en millions RLU par muscle. N = 10 par groupe. Conditions : intensité de champ 200 V/cm, nombre variable d'impulsions de 20 msec, fréquence 1 Hz. | | | | | | | | |
|---|---|---|---|---|---|---|---|---|
| Nombre impulsions | 0 | 1 | 2 | 4 | 6 | 8 | 12 | 16 |
| RLU total | 70 ± 56 | 147 ± 26 | 281 ± 46 | 439 ± 50 | 678 ± 129 | 819 ± 73 | 929 ± 169 | 890 ± 137 |

On observe que l'expression de la luciférase augmente de manière très importante dès l'application d'une seule impulsion, et qu'elle continue d'augmenter en fonction du nombre d'impulsions. Il apparaît ainsi que la variation du nombre d'impulsions délivrées est un moyen de moduler l'efficacité du transfert d'acides nucléiques et d'ajuster le niveau d'expression du transgène.

On confirme également une diminution de la variabilité de la réponse mise en évidence par la diminution de la valeur de la SEM par rapport à la moyenne pour tous les groupes soumis à l'électrotransfert.

### Exemple 14 : effet de l'augmentation de la fréquence des impulsions électriques.

Cet exemple montre que l'augmentation de la fréquence des impulsions permet de manière inattendue d'améliorer l'efficacité de la transfection. D'autre part et dans une perspective clinique, l'augmentation de la fréquence doit améliorer le confort du patient en diminuant la durée totale du traitement.

Cette expérience est réalisée avec des souris C57B1/6. Le plasmide utilisé est le plasmide pXL 2774, la quantité d'ADN administrée est de 15 µg. La fréquence des impulsions électriques est variable (de 0,1 à 4 Hertz). La durée de chaque impulsion est de 20 msec, l'intensité de champ est de 200 Volts/cm, les autres conditions de cette expérience sont celles décrites dans l'exemple 1. Les résultats sont présentés dans le tableau 9.

**Tableau 9 :**

| valeurs moyennes +/- SEM de l'activité luciférase en millions de RLU par muscle. N = 10 pour chaque groupe. Conditions : intensité de champ 200 V/cm, 8 ou 4 impulsions de 20 msec. , fréquence variable. | | | | | | | | |
|---|---|---|---|---|---|---|---|---|
| Fréquence Hertz | 0 | 0.1 | 0,2 | 0,5 | 1 | 2 | 3 | 4 |
| Expérience A 8 impulsions | 5 ±2 | 54 ± 13 | 95 ± 16 | 405 ±60 | 996 ± 156 | 1528 ±257 | | |
| Expérience A 4 impulsions | | 114 ±14 | 163 ±24 | 175 ±26 | 337 ±53 | 587 ± 90 | | |
| Expérience B 8 impulsions | 21 ±14 | | | | 1294 ±189 | 2141 ±387 | 3634 ±868 | 2819 ±493 |
| Expérience B 4 impulsions | | | | | 1451 ±228 | 1572 ±320 | 1222 ±126 | 2474 ±646 |

Les résultats obtenus dans l'expérience « A », tableau 9 montrent que les fréquences plus élevées (≥1 Hz) sont plus efficaces que les fréquences faibles qui correspondent à une durée plus longue entre deux impulsions consécutives (10 secondes à 0.1 Hertz). L'efficacité de la transfection augmente avec la fréquence sur la plage de valeurs testées de 0.1 à 4 Hertz pour 4 impulsions et de 0.1 à 3 Hertz pour 8 impulsions.

### Exemple 15 : effet de l'application d'un champ électrique variant selon une exponentielle décroissante en fonction du temps.

Cette exemple met en évidence l'effet de l'application d'un champ électrique variant selon une exponentielle décroissante sur l'efficacité du transfert d'acides nucléiques.

Cette expérience est réalisée avec des souris C57B1/6.

Le plasmide utilisé est le plasmide pXL 3031. Le plasmide pXL3031 (Figure 12) est un vecteur dérivé du plasmide pXL2774 (WO97/10343) dans lequel le gène luc+ codant pour la luciférase de Photinus pyralis modifiée (cytoplasmique) provenant de pGL3basic (Genbank: CVU47295) a été introduit sous contrôle du promoteur provenant de la région précoce du cytomegalovirus humain (hCMV IE, Genbank HS5IEE) et du signal de poly-adénylation de la région tardive du virus SV40 (Genbank SV4CG). La quantité d'ADN administrée est de 10 µg.

Le générateur d'impulsions électriques utilisé permet de délivrer des impulsions d'une intensité de champ électrique variant selon une exponentielle décroissante en fonction du temps (électropulsateur Equibio, modèle easyjectT plus, Kent UK). Le voltage imposé est le voltage au pic de l'exponentielle. Le deuxième paramètre ajustable est la capacitance (µFarads) qui permet de faire varier la quantité d'énergie délivrée et la constante de temps de l'exponentielle. Les résultats sont présentés dans le tableau 10.

**Tableau 10 :**

| facteur d'augmentation de l'expression (activité luciférase) obtenu par application d'une impulsion à décroissance exponentielle. Le facteur d'augmentation est calculé par référence à l'activité luciférase obtenue avec l'administration du plasmide pXL3031 sans électrotransfert. (valeurs moyennes du facteur d'augmentation, N = 4 à 6 par condition). | | | | | | | |
|---|---|---|---|---|---|---|---|
| | Capa µF 150 | Capa µF 300 | Capa µF 450 | Capa µF 600 | Capa µF 1200 | Capa µF 2400 | Capa µF 3000 |
| 40 V/cm | | | | | | 1,23 | 11 |
| 100 V/cm | | | | 16,5 | 2,8 | 6,5 | 23,9 |
| 150 V/cm | | | | 1,8 | 3,5 | 6,1 | |
| 200 V/cm | | 5,1 | | 15,8 | 18,8 | 121,5 | 189,7 |
| 300 V/cm | 32,1 | 90,5 | 48,7 | 760,4 | 56,2 | | |
| 400 V/cm | | 795 | | | | | |
| 600 V/cm | 62 | | | | | | |
| 800 V/cm | 3,1 | 1,1 | | | | | |

A titre comparatif, le facteur d'augmentation de l'expression obtenu pour le transfert de pXL3031 en présence d'un champ électrique avec des impulsions de formes carrées (intensité de champ de 200 V/cm, 8 impulsions de 20 msec, à une fréquence de 1 Hertz) était de 44 dans la même expérience.

Ces résultats montrent que l'on peut utiliser des impulsions électriques de forme carrée ou d'une intensité décroissant de manière exponentielle en fonction du temps. De plus, dans ce dernier cas, une augmentation importante de l'expression peut être obtenue pour une valeur de champ faible et une capacitance élevée (*e.g.* 200 V/cm, capacitance 3000 µFarad) ou une valeur de champ élevée et une capacitance faible (*e.g*. 400 V/cm, capacitance 300 µFarad).

### Exemple 16 : effet de la combinaison d'une impulsion brève de voltage élevé et de plusieurs impulsions longues de voltage faible.

Cet exemple montre que le champ électrique délivré peut être une combinaison d'au moins un champ compris entre 500 et 800 Volts/cm pendant une courte durée, par exemple 50 ou 100 µsec, et d'au moins un champ faible ( < 100Volts/cm) pendant une durée plus longue, par exemple ≥ 1 msec et jusqu'à 90 msec dans cette expérience.

Les valeurs de champ électrique faible sont ici de 80 V/cm appliquées en 4 impulsions d'une durée de 90 msec avec une fréquence de 1 Hertz. Pour cette expérience deux électropulsateurs sont utilisés. Les impulsions électriques sont appliquées par l'un puis l'autre appareil, le changement s'effectuant en moins d'une seconde à l'aide d'une commande manuelle.

Le plasmide utilisé est le plasmide pXL3031. La quantité d'ADN administrée est de 3 µg. Les valeurs de champ électrique sont indiquées dans le tableau 11 ; les autres conditions de cette expérience sont celles décrites dans l'exemple 1.

**Tableau 11 :**

| valeurs moyenne +/- SEM de l'activité luciférase en millions de RLU par muscle. N = 10 muscles par groupe. | | |
|---|---|---|
| Conditions d'application du champ électrique | Expérience 1 (3 µg pXL3031) | Expérience 2 (3 µg pXL3031) |
| Contrôle (absence de champ électrique) | 320 +/-126 | 75 +/- 27 |
| A1 : 500 V/cm , 1 x 0,1 msec | - | 169 +/- 63 |
| A3 : 800 V/cm , 1 x 0,1 msec | 416 +/- 143 | 272 +/- 84 |
| B : 80 V/cm , 4 x 90 msec | 1282 +/- 203 | 362,21 +/- 85,17 |
| Conditions A1 puis B | - | 1479 +/- 276 |
| Conditions A3 puis B | 3991 +/- 418 | 1426 +/- 209 |
| Conditions B puis A3 | - | 347 +/- 66 |

Le tableau 11, résumant les résultats obtenus pour deux séries d'expériences, montre qu'une brève impulsion de voltage élevé ou que quatre impulsions successives longues et de faible voltage améliorent peu la transfection relativement au groupe contrôle ayant reçu une injection de pXL3031 mais non soumis à un champ électrique. Il en est de même lorsque les impulsions de champ faible sont appliquées avant l'impulsion de champ élevé.

Par contre, dans les deux séries d'expériences, la combinaison d'une brève impulsion de haut voltage suivie de quatre impulsions successives longues et de faible voltage augmente très nettement l'efficacité du transfert de l'ADN.

Les résultats obtenus dans les exemples 1 et 2 ont montré que 8 impulsions de 600, 800 ou 1200 volts d'une durée unitaire de 1 msec à 1 Hz étaient lésionnelles et inhibaient la transfection. Les résultats obtenus dans l'exemple 16 montrent que, dans des conditions particulières, il est possible d'utiliser des intensités de champ de voltage élevées de façon non lésionnelle, en effet d'un point de vu macroscopique les muscles ne sont jamais visiblement altérés. L'utilisation de champs électriques élevés de durée brève combinés à des champs faibles de durée plus longue apparaît comme un moyen supplémentaire de moduler l'efficacité du transfert de l'ADN.

### Exemple 17 : Effet du moment d'injection de l'acide nucléique par rapport à l'application du champ électrique.

Cet exemple illustre le fait que l'acide nucléique peut être administré au moins 30 minutes, et même au moins une heure, avant l'application du champ électrique.

Cette expérience est réalisée avec des souris C57B1/6. Le plasmide utilisé est le plasmide pXL 2774. La quantité d'ADN administrée est de 15 µg ou 1,5 µg. L'injection d'ADN est suivie, ou précédée, de l'application d'un champ électrique dans les conditions suivantes : intensité 200 V/cm , 8 impulsions de 20 msec, fréquence 1 Hz. Les autres conditions de cette expérience sont celles décrites dans l'exemple 1. Un groupe contrôle est constitué d'animaux ayant reçu une injection du plasmide mais n'ayant pas été soumis aux impulsions électriques. Les résultats sont présentés dans le tableau 12

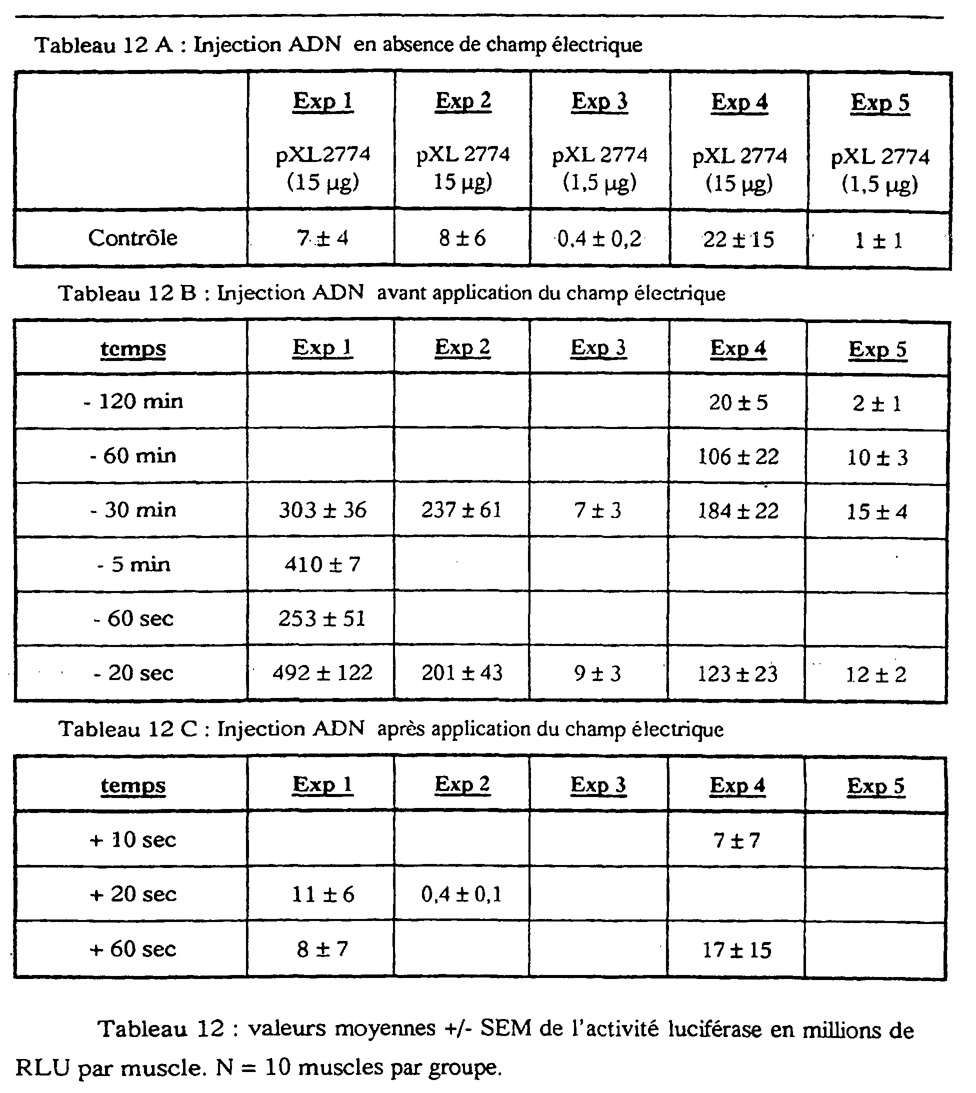

La présence de l'ADN au moment de l'application du champ électrique est une condition de l'efficacité de l'électrotransfection. De façon remarquable, il est observé que l'injection du plasmide peut être réalisée au moins 30 minute et même 1 heure (expériences 4 et 5) avant l'application du champ électrique et ce, sans modification notable du niveau d'expression. Un résultat similaire est obtenu aussi bien avec avec une dose de 15 µg de plasmide par muscle qu'avec une dose 10 fois plus faible de 1,5 µg.

Ces observations permettent notamment d'envisager de multiples injections à des temps variables du même plasmide, ou de différents plasmides, dans le muscle préalablement à l'application du champ électrique. Il est également possible de faire de multiples injections sur une zone étendue du muscle puis d'appliquer une série d'impulsions électriques sur l'ensemble du territoire injecté à traiter.

### Exemple 18 : transfert d'un gène codant pour l'érythropoiëtine (EPO)

Des souris C57B1/6 adultes ont reçu, dans le muscle tibial cranial et de manière unilatérale, une injection de plasmide pXL3348. Le plasmide pXL3348 (Figure 16) est un vecteur dérivé du plasmide pXL2774 dans lequel le gène murin de l'erythropoïetine (NCBI : 193086) a été introduit sous contrôle du promoteur provenant de la région précoce du cytomegalovirus humain (hCMV IE) et du signal de poly-adénylation de la région tardive du virus SV40 (Genbank SV4CG).

Les conditions d'électrotransfert sont les suivantes : intensité champ électrique 200 V/cm, 8 impulsions de 20 msec, fréquence 1 Hz. Le champ électrique est appliqué immédiatement après injection de l'ADN plasmidique.

**Tableau 13 :**

| valeurs moyennes ± SEM. N = 4 à 5. | | | | |
|---|---|---|---|---|
| | Erythropoïetine sérique (mIU/ml) à J7 | | Erythropoïetine sérique (mIU/ml) à J24 | |
| Plasmide | Electrotransfert - | Electrotransfert + | Electrotransfert - | Electrotransfert + |
| pXL3348 (1µg) | 0 | | | 3,0 ± 1,6 , 0 1,12 ± 0,8 |
| pXL3348 (10 µg) | 0,9 ± 0,9 | 61,8 ± 15,8 | 0 | 74,1 ± 28,9 |
| pUC19 (1 µg) | | 0 | | 0 |

| | Hématocrite % Prélèvement à J7 | | Hématocrite % Prélèvement à J24 | |
|---|---|---|---|---|
| Plasmide | Electrotransfert - | Electrotransfert + | Electrotransfert - | Electrotransfert + |
| pXL3348 (1µg) | 38,5 ± 0,5 | 35,0 ± 3,6 | 50,8 ± 2,3 | 81 ± 0,5 |
| pXL3348 (10µg) | 32,0 ± 3,2 | 26,0 ± 4,1 | 69,0 ± 5,1 | 83,0 ± 1,0 |
| PUC 19 (1 µg) | | 30,8 ± 2,3 | | 43,2 ± 0,9 |

On observe, avec l'électrotransfert, une très nette augmentation de la quantité d'érythropoiëtine dans le sang à J7 et J24 pour l'administration de 10 µg de pXL3348. De plus, l'effet physiologique de l'augmentation d'érythropoiëtine qui se traduit par une augmentation de l'hématocrite est très important (85 %), dès J7 et ce, même pour une très faible quantité de plasmide (1 µg).

### Exemple 19 : Effet de l'électrotransfert sur l'expression de transgènes vaccinaux

Cet exemple met en évidence que le procédé selon l'invention est également applicable au transfert de gènes codant pour des polypeptides d'intérêt vaccinal.

L'expérience est réalisée chez des souris Balb/c femelles agées de 9 semaines. Les électrodes utilisées sont des électrodes plates en acier inoxydable distantes de 5 mm. Le VR-HA est un ADN plasmidique comportant le gène de l'hémagglutinine du virus de la grippe (souche A/PR/8/34). Le VR-gB est un ADN plasmidique comportant le gène de la glycoprotéine B (gB) du cytomégalovirus humain (souche Towne).

La solution de plasmide (50 µl d'une solution à 20 µg /ml ou 200 µg /ml dans NaCl 0,9 %) est injectée longitudinalement à travers la peau dans le muscle tibial cranial de manière unilatérale. Les impulsions électriques sont appliquées 20 sec après l'administration du plasmide, perpendiculairement à l'axe du muscle à l'aide d'un générateur d'impulsions carrées ( intensité champ électrique 200 V/cm, 8 impulsions consécutives d'une durée de 20 msec, fréquence 1 Hz).

Pour l'évaluation de la stimulation de la réponse immunitaire, le protocole d'immunisation suivant a été suivi :
- J 0: prélèvement du sérum préimmun
- J 1: primo-injection, plus ou moins électrotransfert
- J 2: prélèvement du sérum immun
- J 2: injection de rappel, plus ou moins électrotransfert
- J 42: prélèvement de sérum immun
- J 63: prélèvement de sérum immun

Les prélèvements sanguins sont effectués au niveau du sinus rétro-orbital. Les dosages des anticorps spécifiques sont réalisés par ELISA. Chaque condition expérimentale est testée sur 10 animaux injectés en unilatéral.

Les résultats concernant les titres en anticorps dirigés contre l'hémagglutinine du virus de la grippe sont présentés dans le tableau 14 A.

**Tableau 14-a :**

| titres en anticorps dirigés contre l'hémagglutinine du virus de la grippe, obtenus après injection de 1 ou 10 µg d'ADN (VR-HA) en absence ou en présence d'impulsions électriques. Les résultats sont les moyennes géométriques de 10 animaux (8 animaux pour le groupe injecté avec 1 µg d'ADN en présence d'impulsions électriques et prélevés à J63) ± écart-type. La valeur de p a été obtenue par comparaison deux à deux des groupes injectés en présence et en absence d'impulsions électriques en utilisant le test non paramétrique de Man-Whitney. | | | | | |
|---|---|---|---|---|---|
| | Electrotransfe rt | J0 | J21 | J42 | J63 |
| VR-HA (1 µg) | - | < 50 | 132 ± 739 | 1201 ± 4380 | 2314 ± 2481 |
| VR-HA (1 µg) | + | < 50 | 1121 ± 1237 | 10441 ± 7819 | 8121 ± 5619 |
| (p) | | | (0,0135) | (0,0022) | (0,0033) |
| VR-HA (10 µg) | - | < 50 | 781 ± 666 | 5113 ± 16015 | 4673 ± 8238 |
| VR-HA (10 µg) | + | < 50 | 4153 ± 2344 | 74761 ± 89228 | 41765 ± 52361 |
| (p) | | | (0,0002) | (0,0005) | (0,0007) |

Ces résultats montrent que les titres en anticorps dirigés contre l'hémagglutinine du virus de la grippe sont augmentés d'un facteur 10 environ dans les groupes soumis aux impulsions électriques. Ainsi les souris ayant reçu 1 µg d'ADN en présence d'impulsions électriques présentent un titre moyen en anticorps légèrement supérieur à celui des souris ayant reçu 10 µg d'ADN en absence d'impulsion électrique.

Les résultats concernant les titres en anticorps dirigés contre la glycoprotéine B du cytomégalovirus humain sont présentés dans le tableau 14 B.

**Tableau 14 B :**

| titres en anticorps dirigés contre la glycoprotéine B (gB) du cytomagalovirus humain obtenus après injection de 10 µg d'ADN (VR-gB) en absence ou en présence d'impulsions électriques. Les résultats sont les moyennes géométriques de 10 animaux (9 animaux pour le groupe injecté en présence d'impulsions électriques) ± écart-type. La valeur de p a été obtenue par comparaison deux à deux des groupes injectés en présence et en absence d'impulsions électriques en utilisant le test non paramétrique de Man-Whitney. | | | | | |
|---|---|---|---|---|---|
| | Electrotransfe rt | J 0 | J 21 | J 42 | J 63 |
| VR-gB (10 µg) | - | < 50 | 73 ± 138 | 755 ± 1766 | 809 ± 1363 |
| VR-gB (10 µg) | + | < 50 | 200 ± 119 | 3057 ± 1747 | 2112 ± 1330 |
| (p) | | | (0,0558) | (0,0108) | (0,0479) |

Ces résultats montrent que les titres en anticorps dirigés contre la glycoprotéine B du cytomégalovirus humain sont augmentés d'un facteur 4 à J42, dans le groupe soumis aux impulsions électriques. On note également que le coefficient de variation est en moyenne trois fois plus faible dans les groupes d'animaux soumis aux impulsions électriques.

## Revendications

1. Utilisation d'acide nucléique pour la préparation d'une composition destinée à être transférée *in vivo* dans des tumeurs, **caractérisée en ce que** ladite composition est à mettre en contact avec les cellules ou tissus, puis est à transférer par application auxdites cellules ou tissus d'une ou plusieurs impulsions électriques d'une intensité comprise entre 1 et 600 volts/cm.

2. Utilisation selon la revendication 1, **caractérisée en ce que** ladite composition est destinée au traitement par thérapie génique.

3. Utilisation selon la revendication 2, **caractérisée en ce que** ladite composition est destinée à une application vaccinale ou immunostimulante.

4. Utilisation selon l'une des revendications 1 à 3, **caractérisée en ce que** la mise en contact est à effectuer par administration directe dans les cellules ou les tissus ou par administration topique ou systématique.

5. Utilisation selon l'une des revendications 1 à 4, **caractérisée en ce que** les cellules des tissus ont des géométries particulières, telles que des cellules de grande taille et/ou de forme allongée et/ou répondant naturellement à des potentiels d'action électriques et/ou ayant une morphologie spécifique.

6. Utilisation selon l'une des revendications 1 à 5, **caractérisée en ce que** l'intensité du champ est comprise entre 4 et 400 volts/cm.

7. Utilisation selon l'une des revendications 1 à 5, **caractérisée en ce que** l'intensité du champ est comprise entre 200 et 600 volts/cm.

8. Utilisation selon l'une des revendications 1 à 5 et 7, **caractérisée en ce que** l'intensité du champ est d'environ 500 volts/cm.

9. Utilisation selon l'une des revendications 1 à 8, **caractérisée en ce que** la durée totale d'application du champ électrique est supérieure à 10 millisecondes.

10. Utilisation selon l'une des revendications 1 à 9, **caractérisée en ce que** l'application au tissu du champ électrique comprend une ou plusieurs impulsions de fréquence régulière.

11. Utilisation selon la revendication 10, **caractérisée en ce que** l'application au tissu du champ électrique comprend entre 1 et 100 000 impulsions de fréquence comprise entre 0,1 et 1000 hertz.

12. Utilisation selon l'une des revendications 1 à 9, **caractérisée en ce que** les impulsions électriques sont délivrées de façon irrégulière les unes par rapport aux autres et **en ce que** la fonction décrivant l'intensité du champ électrique en fonction du temps d'une impulsion est variable.

13. Utilisation selon l'une des revendications 1 à 12, **caractérisée en ce que** l'intégrale de la fonction décrivant la variation du champ électrique avec le temps est supérieure à 1 kVxmsec/cm.

14. Utilisation selon la revendication 13, **caractérisée en ce que** cette intégrale est supérieure ou égale à 5kVxmsec/cm.

15. Utilisation selon l'une des revendications 1 à 14, **caractérisée en ce que** les impulsions électriques sont choisies parmi les impulsions à ondes unipolaires.

16. Utilisation selon l'une des revendications 1 à 14, **caractérisée en ce que** les impulsions électriques sont choisies parmi les impulsions à ondes carrées, les champs électriques générant des ondes à décroissance exponentielle, des ondes unipolaires oscillantes de durée limitée, des ondes bipolaires oscillantes de durée limitée, ou d'autres formes d'ondes.

17. Utilisation selon l'une des revendications 1 à 16, **caractérisée en ce que** les impulsions électriques comprennent des impulsions à ondes carrées.

18. Utilisation selon l'une des revendications 1 à 17, **caractérisée en ce que** les impulsions électriques sont à appliquer avec des électrodes placées de part et d'autre du tissu à traiter ou placées au contact de la peau.

19. Utilisation selon l'une des revendications 1 à 17, **caractérisée en ce que** les impulsions électriques sont à appliquer avec des électrodes introduites à l'intérieur du tissu à traiter.

20. Utilisation selon l'une des revendications 1 à 19, **caractérisée en ce que** l'acide nucléique est à injecter dans le tissu.

21. Utilisation selon l'une des revendications 1 à 19, **caractérisée en ce que** l'acide nucléique est à injecter par voie systémique.

22. Utilisation selon la revendication 21, **caractérisée en ce que** l'acide nucléique est à injecter par voie intra-artérielle ou intra-veineuse.

23. Utilisation selon l'une des revendications 1 à 19, **caractérisée en ce que** l'acide nucléique est à administrer par voie topique, cutanée, orale, vaginale, intranasale, sous cutanée ou intra-oculaire.

24. Utilisation selon l'une des revendications 1 à 23, **caractérisée en ce que** l'acide nucléique est présent dans une composition contenant, en outre, des excipients pharmaceutiquement acceptables pour les différents modes d'administration.

25. Utilisation selon la revendication 24, **caractérisée en ce que** la composition est adaptée à l'administration parentérale.

26. Utilisation selon l'une des revendications 1 à 25, **caractérisée en ce que** l'acide nucléiqué est un acide désoxyribonucléique.

27. Utilisation selon l'une des revendications 1 à 25, **caractérisée en ce que** l'acide nucléique est un acide ribonucléique.

28. Utilisation selon l'une des revendications 1 à 27, **caractérisée en ce que** l'acide nucléique est d'origine synthétique ou biosynthétique, ou extrait d'un virus ou d'un organisme procaryote ou eucaryote unicellulaire ou pluricellulaire.

29. Utilisation selon la revendication 28, **caractérisée en ce que** l'acide nucléique administré est associé à tout ou partie des composants de l'organisme d'origine et/ou du système de synthèse.

30. Utilisation selon l'une des revendications 1 à 29, **caractérisée en ce que** l'acide nucléique code pour un ARN ou une protéine d'intérêt.

31. Utilisation selon la revendication 30, **caractérisée en ce que** l'ARN est un ARN catalytique ou antisens.

32. Utilisation selon la revendication 30, **caractérisée en ce que** l'acide nucléique code pour une protéine choisie parmi les enzymes, les dérivés sanguins, les hormones, les lymphokines, les cytokines, les facteurs de croissance, les facteurs trophiques, les facteurs angiogéniques, les facteurs neurotrophiques, les facteurs de croissance osseuse, les facteurs hématopoiétiques, les facteurs de coagulation, les antigènes et les protéines impliquées dans le métabolisme des acides aminés, des lipides et des autres constituants essentiels de la cellule.

33. Utilisation selon la revendication 32, **caractérisée en ce que** l'acide nucléique code pour les facteurs angiogéniques VEGF et FGF, les facteurs neurotrophiques BDNF, CNTF, NGF, IGF, GMF, aFGF, NT3, NT5, la protéine Gax, l'hormone de croissance,l'α-1-antitrypsine, la calcitonine, la leptine et les apolipoprotéines, les enzymes de biosynthèse des vitamines, des hormones et des neuromédiateurs.

34. Utilisation selon la revendication 30, **caractérisée en ce que** l'acide nucléique code pour un anticorps, un fragment variable d'anticorps simple chaîne (ScFv) ou tout autre fragment d'anticorps possédant des capacités de reconnaissance dans un but d'immunothérapie, ou code pour un récepteur soluble, pour un peptide agoniste ou antagoniste d'un récepteur ou d'une protéine d'adhésion, pour une protéine artificielle, chimérique ou tronquée.

35. Utilisation selon la revendication 34, **caractérisée en ce que** l'acide nucléique code pour un anticorps antiidiotype, un fragment soluble du récepteur CD4 ou du récepteur du TNFa ou du récepteur de l'acétylcholine.

36. Utilisation selon l'une des revendications 32 à 35, **caractérisée en ce que** l'acide nucléique code pour un précurseur d'une protéine thérapeutique.

37. Utilisation selon l'une des revendications 1 à 36, **caractérisée en ce que** l'acide nucléique est sous forme d'un plasmide.

38. Utilisation selon l'une des revendications 1 à 36, **caractérisée en ce que** l'acide nucléique contient un gène de grande taille et/ou des introns et/ou des éléments régulateurs de petite ou de grande taille.

39. Utilisation selon l'une des revendications 1 à 36, **caractérisée en ce que** l'acide nucléique est un ADN épisomal ou un chromosome artificiel de levure ou un minichromosome.

40. Utilisation selon l'une des revendications 1 à 39, **caractérisée en ce que** l'acide nucléique contient des séquences permettant et/ou favorisant l'expression du transgène dans le tissu.

41. Utilisation selon l'une des revendications 1 à 40, **caractérisée en ce que** l'acide est associé à tout type de vecteurs ou à toute combinaison de vecteurs permettant d'améliorer le transfert d'acide nucléique tels que des virus, des agents synthétiques ou biosynthétiques, ou encore des billes propulsées ou non.

42. Utilisation selon l'une des revendications 1 à 41, **caractérisée en ce que** le tissu est soumis à un traitement visant à améliorer le transfert de gène, un traitement de nature pharmacologique en application locale ou systémique, ou un traitement enzymatique, perméabilisant, chirurgical, mécanique, thermique ou physique.

43. Utilisation selon l'une des revendications 1 à 41, **caractérisée en ce qu'**elle permet de faire produire par le tissu un agent à des doses physiologiques et/ou thérapeutiques, soit dans les cellules musculaires, soit sécrété.

44. Utilisation selon l'une des revendications 1 à 43, **caractérisée en ce qu'**elle permet de moduler la quantité de transgène exprimé en modulant le volume de tissu transfecté.

45. Utilisation selon la revendication 44, **caractérisée en ce qu'**elle permet de moduler le volume de tissu transfecté par l'utilisation de sites multiples d'administration.

46. Utilisation selon l'une des revendications 1 à 45, **caractérisée en ce qu'**elle permet de moduler la quantité de transgène exprimé en modulant le nombre, la forme, la surface et la disposition des électrodes, et en variant l'intensité de champ, le nombre, la durée, la fréquence et la forme des impulsions, ainsi que la quantité et le volume d'administration de l'acide nucléique.

47. Utilisation selon l'une des revendications 1 à 46, **caractérisée en ce qu'**elle permet de contrôler la localisation des tissus à transfecter par le volume de tissu soumis aux impulsions électriques locales.

48. Utilisation selon l'une des revendications 1 à 47, **caractérisée en ce qu'**elle - permet un retour à la situation initiale par l'ablation de la zone de tissu transfectée.

49. Acide nucléique et un système produisant un champ électrique d'une intensité comprise entre 1 et 600 Volts/cm, comme produit de combinaison pour leur administration séparée ou étalée dans le temps *in vivo* à une tumeur et, pour la thérapie génique reposant sur l'électrotransfection *in vivo* dans les tissus après administration de l'acide nucléique.

50. Produit de combinaison selon la revendication 49, **caractérisé en ce que** l'intensité du champ est comprise entre 200 et 600 volts/cm.

51. Produit de combinaison selon la revendication 49, **caractérisé en ce que** l'intensité du champ est d'environ 500 volts/cm.

52. Produit de combinaison selon l'une des revendications 49 à 51, **caractérisé en ce que** la durée totale d'application du champ électrique est supérieure à 10 millisecondes.

53. Produit de combinaison selon l'une des revendications 49 à 52, **caractérisé en ce que** l'application au tissu du champ électrique comprend une ou plusieurs impulsions de fréquence régulière.

54. Produit de combinaison selon la revendication 53, **caractérisé en ce que** l'application au tissu du champ électrique comprend entre 1 et 100 000 impulsions de fréquence comprise entre 0,1 et 1000 hertz.

55. Produit de combinaison selon l'une des revendications 49 à 52, **caractérisé en ce que** les impulsions électriques sont délivrées de façon irrégulière les unes par rapport aux autres et **en ce que** la fonction décrivant l'intensité du champ électrique en fonction du temps d'une impulsion est variable.

56. Produit de combinaison selon l'une des revendications 49 à 55, **caractérisé en ce que** l'intégrale de la fonction décrivant la variation du champ électrique avec le temps est supérieure à 1 kVxmsec/cm.

57. Produit de combinaison selon la revendication 56, **caractérisé en ce que** cette intégrale est supérieure ou égale à 5 kVxmsec/cm.

58. Produit de combinaison selon l'une des revendications 49 à 57, **caractérisé en ce que** les impulsions électriques sont choisies parmi les impulsions à ondes carrées, les champs électriques générant des ondes à décroissance exponentielle, des ondes unipolaires oscillantes de durée limitée, des ondes bipolaires oscillantes de durée limitée, ou d'autres formes d'ondes.

59. Produit de combinaison selon l'une des revendications 49 à 58, **caractérisé en ce que** les impulsions électriques comprennent des impulsions à ondes carrées.

60. Produit de combinaison selon l'une des revendications 49 à 59, **caractérisé en ce que** les impulsions électriques sont à appliquer de façon externe.

61. Produit de combinaison selon l'une des revendications 49 à 59, **caractérisé en ce que** les impulsions électriques sont à appliquer à l'intérieur du tissu.

62. Produit de combinaison selon l'une des revendications 49 à 61, **caractérisé en ce que** l'acide nucléique est à injecter dans le tissu.

63. Produit de combinaison selon l'une des revendications 49 à 61, **caractérisé en ce que** l'acide nucléique est à injecter par voie systémique.

64. Produit de combinaison selon la revendication 63, **caractérisé en ce que** l'acide nucléique est à injecter par voie intra-artérielle ou intra-veineuse.

65. Produit de combinaison selon l'une des revendications 49 à 61, **caractérisé en ce que** l'acide nucléique est à administrer par voie topique, cutanée, orale, vaginale, intranasale, intramusculaire, sous cutanée ou intra-oculaire.

66. Produit de combinaison selon l'une des revendications 49 à 65, **caractérisé en ce que** l'acide nucléique est présent dans une composition contenant, en outre, des excipients pharmaceutiquement acceptables pour les différents modes d'administration.

67. Produit de combinaison selon la revendication 66, **caractérisé en ce que** la composition est adaptée à l'administration parentérale.

68. Produit de combinaison selon l'une des revendications 49 à 67, **caractérisé en ce que** l'acide nucléique est un acide désoxyribonucléique.

69. Produit de combinaison selon l'une des revendications 49 à 67, **caractérisé en ce que** l'acide nucléique est un acide ribonucléique.

70. Produit de combinaison selon l'une des revendications 49 à 69, **caractérisé en ce que** l'acide nucléique est d'origine synthétique ou biosynthétique, ou extrait d'un virus ou d'un organisme procaryote ou eucaryote unicellulaire ou pluricellulaire.

71. Produit de combinaison selon la revendication 70, **caractérisé en ce que** l'acide nucléique administré est associé à tout ou partie des composants de l'organisme d'origine et/ou du système de synthèse.

72. Produit de combinaison selon l'une des revendications 49 à 71, **caractérisé en ce que** l'acide nucléique code pour un ARN ou une protéine d'intérêt.

73. Produit de combinaison selon la revendication 72, **caractérisé en ce que** l'ARN est un ARN catalytique ou antisens.

74. Produit de combinaison selon la revendication 72, **caractérisé en ce que** l'acide nucléique code pour une protéine choisie parmi les enzymes, les dérivés sanguins, les hormones, les lymphokines, les facteurs de croissance, les facteurs trophiques, les facteurs angiogéniques, les facteurs neurotrophiques, les facteurs de croissance osseuse, les facteurs hématopoïétiques, les facteurs de coagulation, les antigènes et les protéines impliquées dans le métabolisme des acides aminés, des lipides et des autres constituants essentiels de la cellule.

75. Produit de combinaison selon la revendication 74, **caractérisé en ce que** l'acide nucléique code pour les facteurs angiogéniques VEGF et FGF, les facteurs neurotrophiques BDNF, CNTF, NGF, IGF, GMF, aFGF, NT3, NT5, la protéine Gax, l'hormone de croissance, une cytokine,l'α-1-antitrypsine, la calcitonine, la leptine et les apolipoprotéines, les enzymes de biosynthèse des vitamines, des hormones et des neuromédiateurs.

76. Produit de combinaison selon la revendication 72, **caractérisé en ce que** l'acide nucléique code pour un anticorps, un fragment variable d'anticorps simple chaîne (ScFv) ou tout autre fragment d'anticorps possédant des capacités de reconnaissance dans un but d'immunothérapie, ou code pour un récepteur soluble, pour un peptide agoniste ou antagoniste d'un récepteur ou d'une protéine d'adhésion, pour une protéine artificielle, chimérique ou tronquée.

77. Produit de combinaison selon la revendication 76, **caractérisé en ce que** l'acide nucléique code pour un anticorps antiidiotype, un fragment soluble du récepteur CD4 ou du récepteur du TNFa ou du récepteur de l'acétylcholine.

78. Produit de combinaison selon l'une des revendications 74 à 77, **caractérisé en ce que** l'acide nucléique code pour un précurseur d'une protéine thérapeutique.

79. Produit de combinaison selon l'une des revendications 49 à 78, **caractérisé en ce que** l'acide nucléique est sous forme d'un plasmide.

80. Produit de combinaison selon l'une des revendications 49 à 78, **caractérisé en ce que** l'acide nucléique contient un gène de grande taille et/ou des introns et/ou des éléments. régulateurs de petite ou de grande taille.

81. Produit de combinaison selon l'une des revendications 49 à 78, **caractérisé en ce que** l'acide nucléique est un ADN épisomal ou un chromosome artificiel de levure ou un minichromosome.

82. Produit de combinaison selon l'une des revendications 49 à 81, **caractérisé en ce que** l'acide nucléique contient des séquences permettant et/ou favorisant l'expression du transgène dans le tissu.

83. Produit de combinaison selon l'une des revendications 49 à 82, **caractérisé en ce que** l'acide est associé à tout type de vecteurs ou à toute combinaison de vecteurs permettant d'améliorer le transfert d'acide nucléique tels que des virus, des agents synthétiques ou biosynthétiques, ou encore des billes propulsées ou non.

84. Produit de combinaison selon l'une des revendications 49 à 83, **caractérisé en ce que** le tissu est soumis à un traitement visant à améliorer le transfert de gène, un traitement de nature pharmacologique en application locale ou systémique, ou un traitement enzymatique, perméabilisant, chirurgical, mécanique, thermique ou physique.

85. Produit de combinaison selon l'une des revendications 49 à 84, **caractérisé en ce qu'**il permet de faire produire par le tissu un agent à des doses physiologiques et/ou thérapeutiques, soit dans les cellules du tissu, soit secrété.

86. Produit de combinaison selon l'une des revendications 49 à 84, **caractérisé en ce qu'**il permet de moduler la quantité de transgène exprimé en modulant le volume de tissu transfecté.

87. Produit de combinaison selon la revendication 86, **caractérisé en ce qu'**il permet de moduler le volume de tissu transfecté par l'utilisation de sites multiples d'administration.

88. Produit de combinaison selon l'une des revendications 49 à 87, **caractérisé en ce qu'**il permet de moduler la quantité de transgène exprimé en modulant le nombre, la forme, la surface et la disposition des électrodes, et en variant l'intensité de champ, le nombre, la durée, la fréquence et la forme des impulsions, ainsi que la quantité et le volume d'administration de l'acide nucléique.

89. Produit de combinaison selon l'une des revendications 49 à 88, **caractérisé en ce qu'**il permet de contrôler la localisation des tissus transfectés par le volume de tissu soumis aux impulsions électriques locales.

90. Produit de combinaison selon l'une des revendications 49 à 89, **caractérisé en ce qu'**il permet un retour à la situation initiale par l'ablation de la zone de tissu transféctée.

## Claims

1. Use of nucleic acid for preparing a composition intended to be transferred *in vivo* into tumours, **characterized in that** the said composition is to be brought into contact with the cells or tissues, and is then to be transferred by applying to the said cells or tissues one or more electrical pulses of an intensity of between 1 and 600 volts/cm.

2. Use according to Claim 1, **characterized in that** the said composition is intended for treatment by gene therapy.

3. Use according to Claim 2, **characterized in that** the said composition is intended for a vaccinal or immunostimulant application.

4. Use according to one of Claims 1 to 3, **characterized in that** the bringing into contact is to be carried out by direct administration into the cells or the tissues or by topical or systemic administration.

5. Use according to one of Claims 1 to 4, **characterized in that** the cells of the tissues have specific geometries, such as cells of large size and/or elongated shape and/or naturally responding to electrical action potentials and/or having a specific morphology.

6. Use according to one of Claims 1 to 5, **characterized in that** the intensity of the field is between 4 and 400 volts/cm.

7. Use according to one of Claims 1 to 5, **characterized in that** the intensity of the field is between 200 and 600 volts/cm.

8. Use according to one of Claims 1 to 5 and 7, **characterized in that** the intensity of the field is approximately 500 volts/cm.

9. Use according to one of Claims 1 to 8, **characterized in that** the total duration of application of the electric field is greater than 10 milliseconds.

10. Use according to one of Claims 1 to 9, **characterized in that** the application, to the tissue, of the electric field comprises one or more pulses of regular frequency.

11. Use according to Claim 10, **characterized in that** the application, to the tissue, of the electric field comprises between 1 and 100 000 pulses of frequency between 0.1 and 1000 hertz.

12. Use according to one of Claims 1 to 9, **characterized in that** the electrical pulses are delivered in an irregular manner relative to each other and **in that** the function describing the intensity of the electric field as a function of the time for one pulse is variable.

13. Use according to one of Claims 1 to 12, **characterized in that** the integral of the function describing the variation of the electric field with time is greater than 1 kV × msec/cm.

14. Use according to Claim 13, **characterized in that** this integral is greater than or equal to 5 kV x msec/cm.

15. Use according to one of Claims 1 to 14, **characterized in that** the electrical pulses are chosen from unipolar wave pulses.

16. Use according to one of Claims 1 to 14, **characterized in that** the electrical pulses are chosen from square wave pulses, electric fields generating exponentially decreasing waves, oscillating unipolar waves of limited duration, oscillating bipolar waves of limited duration, or other wave forms.

17. Use according to one of Claims 1 to 16, **characterized in that** the electrical pulses comprise square wave pulses.

18. Use according to one of Claims 1 to 17, **characterized in that** the electrical pulses are to be applied using electrodes placed on either side of the tissue to be treated or placed in contact with the skin.

19. Use according to one of Claims 1 to 17, **characterized in that** the electrical pulses are to be applied using electrodes introduced inside the tissue to be treated.

20. Use according to one of Claims 1 to 19, **characterized in that** the nucleic acid is to be injected into the tissue.

21. Use according to one of Claims 1 to 19, **characterized in that** the nucleic acid is to be injected by the systemic route.

22. Use according to Claim 21, **characterized in that** the nucleic acid is to be injected by the intra-arterial or intravenous route.

23. Use according to one of Claims 1 to 19, **characterized in that** the nucleic acid is to be administered by the topical, cutaneous, oral, vaginal, intranasal, subcutaneous or intraocular route.

24. Use according to one of Claims 1 to 23, **characterized in that** the nucleic acid is present in a composition containing, in addition, pharmaceutically acceptable excipients for the different modes of administration.

25. Use according to Claim 24, **characterized in that** the composition is suitable for parenteral administration.

26. Use according to one of Claims 1 to 25, **characterized in that** the nucleic acid is a deoxyribonucleic acid.

27. Use according to one of Claims 1 to 25, **characterized in that** the nucleic acid is a ribonucleic acid.

28. Use according to one of Claims 1 to 27, **characterized in that** the nucleic acid is of synthetic or biosynthetic origin, or extracted from a virus or from a unicellular or pluricellular eukaryotic or prokaryotic organism.

29. Use according to Claim 28, **characterized in that** the nucleic acid administered is combined with all or part of the components of the organism of origin and/or of the synthesis system.

30. Use according to one of Claims 1 to 29, **characterized in that** the nucleic acid encodes an RNA or a protein of interest.

31. Use according to Claim 30, **characterized in that** the RNA is a catalytic or antisense RNA.

32. Use according to Claim 30, **characterized in that** the nucleic acid encodes a protein chosen from enzymes, blood derivatives, hormones, lymphokines, cytokines, growth factors, trophic factors, angiogenic factors, neurotrophic factors, bone growth factors, haematopoietic factors, coagulation factors, antigens and proteins involved in the metabolism of amino acids, lipids and other essential constituents of the cell.

33. Use according to Claim 32, **characterized in that** the nucleic acid encodes the angiogenic factors VEGF and FGF, the neurotrophic factors BDNF, CNTF, NGF, IGF, GMF, aFGF, NT3, NT5, the Gax protein, growth hormone, α-1-antitrypsin, calcitonin, leptin and the apolipoproteins, and the enzymes for the biosynthesis of vitamins, hormones and neuromediators.

34. Use according to Claim 30, **characterized in that** the nucleic acid encodes an antibody, a variable fragment of a single-chain antibody (ScFv) or any other antibody fragment having recognition capacities for the purposes of immunotherapy, or encodes a soluble receptor, a peptide which is an agonist or antagonist of a receptor or of adhesion protein, or an artificial, chimeric or truncated protein.

35. Use according to Claim 34, **characterized in that** the nucleic acid encodes an antiidiotype antibody, or a soluble fragment of the CD4 receptor or of the TNFα receptor or of the acetylcholine receptor.

36. Use according to one of Claims 32 to 35, **characterized in that** the nucleic acid encodes a precursor of a therapeutic protein.

37. Use according to one of Claims 1 to 36, **characterized in that** the nucleic acid is in the form of a plasmid.

38. Use according to one of Claims 1 to 36, **characterized in that** the nucleic acid contains a gene of large size and/or introns and/or regulatory elements of small or large size.

39. Use according to one of Claims 1 to 36, **characterized in that** the nucleic acid is an episomal DNA or a yeast artificial chromosome or a minichromosome.

40. Use according to one of Claims 1 to 39, **characterized in that** the nucleic acid contains sequences allowing and/or promoting the expression of the transgene in the tissue.

41. Use according to one of Claims 1 to 40, **characterized in that** the acid is combined with any type of vectors or with any combination of vectors which make it possible to improve the transfer of nucleic acid, such as viruses, synthetic or biosynthetic agents, or beads which are propelled or otherwise.

42. Use according to one of Claims 1 to 41, **characterized in that** the tissue is subjected to a treatment intended to improve gene transfer, a treatment of pharmacological nature in the form of a local or systemic application, or an enzymatic, permeabilizing, surgical, mechanical, thermal or physical treatment.

43. Use according to one of Claims 1 to 41, **characterized in that** it makes it possible to cause the tissue to produce an agent at physiological and/or therapeutic doses, either in the muscle cells, or secreted.

44. Use according to one of Claims 1 to 43, **characterized in that** it makes it possible to modulate the quantity of transgene expressed by modulating the volume of tissue transfected.

45. Use according to Claim 44, **characterized in that** it makes it possible to modulate the volume of tissue transfected by the use of multiple sites of administration.

46. Use according to one of Claims 1 to 45, **characterized in that** it makes it possible to modulate the quantity of transgene expressed by modulating the number, shape, surface and arrangement of the electrodes, and by varying the field intensity, the number, the duration, the frequency and the form of the pulses, and also the quantity and the volume of the nucleic acid for administration.

47. Use according to one of Claims 1 to 46, **characterized in that** it makes it possible to control the localization of the tissues to be transfected by means of the volume of tissue subjected to the local electrical pulses.

48. Use according to one of Claims 1 to 47, **characterized in that** it allows a return to the initial situation by removal of the transfected tissue area.

49. Nucleic acid and a system producing an electric field of an intensity between 1 and 600 volts/cm, as combination product for their administration separately or spaced out over time, *in vivo*, to a tumour, and for gene therapy based on *in vivo* electrotransfection into the tissues after administration of the nucleic acid.

50. Combination product according to Claim 49, **characterized in that** the field intensity is between 200 and 600 volts/cm.

51. Combination product according to Claim 49, **characterized in that** the field intensity is approximately 500 volts/cm.

52. Combination product according to one of Claims 49 to 51, **characterized in that** the total duration of application of the electric field is greater than 10 milliseconds.

53. Combination product according to one of Claims 49 to 52, **characterized in that** the application, to the tissue, of the electric field comprises one or more pulses of regular frequency.

54. Combination product according to Claim 53, **characterized in that** the application, to the tissue, of the electric field comprises between 1 and 100 000 pulses of frequency between 0.1 and 1000 hertz.

55. Combination product according to one of Claims 49 to 52, **characterized in that** the electrical pulses are delivered in an irregular manner relative to one another and **in that** the function describing the intensity of the electric field as a function of the time for one pulse is variable.

56. Combination product according to one of Claims 49 to 55, **characterized in that** the integral of the function describing the variation of the electric field with time is greater than 1 kV × msec/cm.

57. Combination product according to Claim 56, **characterized in that** this integral is greater than or equal to 5 kV × msec/cm.

58. Combination product according to one of Claims 49 to 57, **characterized in that** the electrical pulses are chosen from square wave pulses, electric fields generating exponentially decreasing waves, oscillating unipolar waves of limited duration, oscillating bipolar waves of limited duration, or other wave forms.

59. Combination product according to one of Claims 49 to 58, **characterized in that** the electrical pulses comprise square wave pulses.

60. Combination product according to one of Claims 49 to 59, **characterized in that** the electrical pulses are to be applied externally.

61. Combination product according to one of Claims 49 to 59, **characterized in that** the electrical pulses are to be applied inside the tissue.

62. Combination product according to one of Claims 49 to 61, **characterized in that** the nucleic acid is to be injected into the tissue.

63. Combination product according to one of Claims 49 to 61, **characterized in that** the nucleic acid is to be injected by the systemic route.

64. Combination product according to Claim 63, **characterized in that** the nucleic acid is to be injected by the intra-arterial or intravenous route.

65. Combination product according to one of Claims 49 to 61, **characterized in that** the nucleic acid is to be administered by the topical, cutaneous, oral, vaginal, intranasal, intramuscular, subcutaneous or intraocular route.

66. Combination product according to one of Claims 49 to 65, **characterized in that** the nucleic acid is present in a composition containing, in addition, pharmaceutically acceptable excipients for the different modes of administration.

67. Combination product according to Claim 66, **characterized in that** the composition is suitable for parenteral administration.

68. Combination product according to one of Claims 49 to 67, **characterized in that** the nucleic acid is a deoxyribonucleic acid.

69. Combination product according to one of Claims 49 to 67, **characterized in that** the nucleic acid is a ribonucleic acid.

70. Combination product according to one of Claims 49 to 69, **characterized in that** the nucleic acid is of synthetic or biosynthetic origin, or extracted from a virus or a unicellular or pluricellular eukaryotic or prokaryotic organism.

71. Combination product according to Claim 70, **characterized in that** the nucleic acid administered is combined with all or part of the components of the organism of origin and/or of the synthesis system.

72. Combination product according to one of Claims 49 to 71, **characterised in that** the nucleic acid encodes an RNA or a protein of interest.

73. Combination product according to Claim 72, **characterized in that** the RNA is a catalytic or antisense RNA.

74. Combination product according to Claim 72, **characterized in that** the nucleic acid encodes a protein chosen from enzymes, blood derivatives, hormones, lymphokines, growth factors, trophic factors, angiogenic factors, neurotrophic factors, bone growth factors, haematopoietic factors, coagulation factors, antigens and proteins involved in the metabolism of amino acids, lipids and other essential constituents of the cell.

75. Combination product according to Claim 74, **characterized in that** the nucleic acid encodes the angiogenic factors VEGF and FGF, the neurotrophic factors BDNF, CNTF, NGF, IGF, GMF, aFGF, NT3, NT5, the Gax protein, growth hormone, a cytokine, α-1-antitrypsin, calcitonin, leptin and the apolipoproteins, and the enzymes for the biosynthesis of vitamins, hormones and neuromediators.

76. Combination product according to Claim 72, **characterized in that** the nucleic acid encodes an antibody, a variable fragment of a single-chain antibody (ScFv) or any other antibody fragment possessing recognition capacities for the purposes of immunotherapy, or encodes a soluble receptor, a peptide which is an agonist or antagonist of a receptor or of an adhesion protein, or an artificial, chimeric or truncated protein.

77. Combination product according to Claim 76, **characterized in that** the nucleic acid encodes an antiidiotype antibody, or a soluble fragment of the CD4 receptor or of the TNFα receptor or of the acetylcholine receptor.

78. Combination product according to one of Claims 74 to 77, **characterized in that** the nucleic acid encodes a precursor of a therapeutic protein.

79. Combination product according to one of Claims 49 to 78, **characterized in that** the nucleic acid is in the form of a plasmid.

80. Combination product according to one of Claims 49 to 78, **characterized in that** the nucleic acid contains a gene of large size and/or introns and/or regulatory elements of small or large size.

81. Combination product according to one of Claims 49 to 78, **characterized in that** the nucleic acid is an episomal DNA or a yeast artificial chromosome or a minichromosome.

82. Combination product according to one of Claims 49 to 81, **characterized in that** the nucleic acid contains sequences allowing and/or promoting the expression of the transgene in the tissue.

83. Combination product according to one of Claims 49 to 82, **characterized in that** the acid is combined with any type of vectors or with any combination of vectors which make it possible to improve the transfer of nucleic acid, such as viruses, synthetic or biosynthetic agents, or beads which are propelled or otherwise.

84. Combination product according to one of Claims 49 to 83, **characterized in that** the tissue is subjected to a treatment intended to improve gene transfer a treatment of pharmacological nature in the form of a local or systemic application, or an enzymatic, permeabilizing, surgical, mechanical, thermal or physical treatment.

85. Combination product according to one of Claims 49 to 84, **characterized in that** it makes it possible to cause the tissue to produce an agent at physiological and/or therapeutic doses, either in the cells of the tissue, or secreted.

86. Combination product according to one of Claims 49 to 84, **characterized in that** it makes it possible to modulate the quantity of transgene expressed by modulating the volume of tissue transfected.

87. Combination product according to Claim 86, **characterized in that** it makes it possible to modulate the volume of tissue transfected by the use of multiple sites of administration.

88. Combination product according to one of Claims 49 to 87, **characterized in that** it makes it possible to modulate the quantity of transgene expressed by modulating the number, shape, surface and arrangement of the electrodes, and by varying the field intensity, the number, the duration, the frequency and the form of the pulses, and also the quantity and the volume of nucleic acid for administration.

89. Combination product according to one of Claims 49 to 88, **characterized in that** it makes it possible to control the localization of the tissues transfected by means of the volume of tissue subjected to the local electrical pulses.

90. Combination product according to one of Claims 49 to 89, **characterized in that** it allows a return to the initial situation by removal of the transfected tissue area.

## Patentansprüche

1. Verwendung von Nukleinsäure für die Herstellung einer Zusammensetzung, welche dazu bestimmt ist, *in vivo* in Tumore transferiert zu werden, **dadurch gekennzeichnet, dass** die Zusammensetzung mit den Zellen oder Geweben in Kontakt gebracht werden soll, dann durch Anwendung von einem oder mehreren elektrischen Impuls(en) mit einer Intensität zwischen 1 und 600 Volt/cm eingeschlossen auf die Zellen oder Gewebe transferiert werden soll.

2. Verwendung nach Anspruch 1, **dadurch gekennzeichnet, dass** die Zusammensetzung für die Behandlung durch Gentherapie bestimmt ist.

3. Verwendung nach Anspruch 2, **dadurch gekennzeichnet, dass** die Zusammensetzung für eine Impfanwendung oder immunstimulierende Anwendung bestimmt ist.

4. Verwendung nach einem der Ansprüche 1 bis 3, **dadurch gekennzeichnet, dass** das Inkontaktbringen durch direkte Verabreichung in die Zellen oder die Gewebe oder durch topische oder systemische Verabreichung erfolgt.

5. Verwendung nach einem der Ansprüche 1 bis 4, **dadurch gekennzeichnet, dass** die Zellen der Gewebe besondere Geometrien haben, wie Zellen von großer Größe und/oder von länglicher Form und/oder welche von Natur aus auf elektrische Aktionspotentiale ansprechen und/oder welche eine spezifische Morphologie aufweisen.

6. Verwendung nach einem der Ansprüche 1 bis 5, **dadurch gekennzeichnet, dass** die Feldstärke zwischen 4 und 400 Volt/cm eingeschlossen liegt.

7. Verwendung nach einem der Ansprüche 1 bis 5, **dadurch gekennzeichnet, dass** die Feldstärke zwischen 200 und 600 Volt/cm eingeschlossen liegt.

8. Verwendung nach einem der Ansprüche 1 bis 5 und 7, **dadurch gekennzeichnet, dass** die Feldstärke ungefähr 500 Volt/cm beträgt.

9. Verwendung nach einem der Ansprüche 1 bis 8, **dadurch gekennzeichnet, dass** die gesamte Anwendungsdauer des elektrischen Felds über 10 Millisekunden liegt.

10. Verwendung nach einem der Ansprüche 1 bis 9, **dadurch gekennzeichnet, dass** die Anwendung des elektrischen Felds auf das Gewebe einen oder mehrere Impulse von regelmäßiger Frequenz umfasst.

11. Verwendung nach Anspruch 10, **dadurch gekennzeichnet, dass** die Anwendung des elektrischen Felds auf das Gewebe zwischen 1 und 100000 Impulse mit einer Frequenz zwischen 0,1 und 1000 Hertz umfasst.

12. Verwendung nach einem der Ansprüche 1 bis 9, **dadurch gekennzeichnet, dass** die elektrischen Impulse auf unregelmäßige Weise bezogen aufeinander abgegeben werden und dass die Funktion, welche die Stärke des elektrischen Felds abhängig von dem Zeitpunkt eines Impulses beschreibt, variabel ist.

13. Verwendung nach einem der Ansprüche 1 bis 12, **dadurch gekennzeichnet, dass** das Integral der Funktion, welche die Variation des elektrischen Felds mit der Zeit beschreibt, über 1 kV·ms/cm liegt.

14. Verwendung nach Anspruch 13, **dadurch gekennzeichnet, dass** dieses Integral größer oder gleich 5 kV·ms/cm beträgt

15. Verwendung nach einem der Ansprüche 1 bis 14, **dadurch gekennzeichnet, dass** die elektrischen Impulse ausgewählt werden aus den Impulsen mit unipolaren Wellen.

16. Verwendung nach einem der Ansprüche 1 bis 14, **dadurch gekennzeichnet, dass** die elektrischen Impulse ausgewählt werden aus den Impulsen mit Rechteckwellen, den elektrischen Feldem, welche Wellen mit exponentieller Abnahme, unipolare oszillierende Wellen von begrenzter Dauer, bipolare oszillierende Wellen von begrenzter Dauer oder andere Wellenformen erzeugen.

17. Verwendung nach einem der Ansprüche 1 bis 16, **dadurch gekennzeichnet, dass** die elektrischen Impulse Impulse mit Rechteckwellen umfassen.

18. Verwendung nach einem der Ansprüche 1 bis 17, **dadurch gekennzeichnet, dass** die elektrischen Impulse anzuwenden sind mit Elektroden, welche auf beiden Seiten des zu behandelnden Gewebes angeordnet sind oder in Kontakt mit der Haut angeordnet sind.

19. Verwendung nach einem der Ansprüche 1 bis 17, **dadurch gekennzeichnet, dass** die elektrischen Impulse anzuwenden sind mit Elektroden, die in das Innere des zu behandelnden Gewebes eingeführt sind.

20. Verwendung nach einem der Ansprüche 1 bis 19, **dadurch gekennzeichnet, dass** die Nukleinsäure in das Gewebe zu injizieren ist.

21. Verwendung nach einem der Ansprüche 1 bis 19, **dadurch gekennzeichnet, dass** die Nukleinsäure systemisch zu injizieren ist.

22. Verwendung nach Anspruch 21, **dadurch gekennzeichnet, dass** die Nukleinsäure auf intraarteriellem oder intravenösem Wege zu injizieren ist.

23. Verwendung nach einem der Ansprüche 1 bis 19, **dadurch gekennzeichnet, dass** die Nukleinsäure auf topischem, kutanem, oralem, vaginalem, intranasalem, subkutanem oder intraokularem Wege zu verabreichen ist.

24. Verwendung nach einem der Ansprüche 1 bis 23, **dadurch gekennzeichnet, dass** die Nukleinsäure in einer Zusammensetzung vorliegt, welche außerdem pharmazeutisch annehmbare Vehikei für die verschiedehen Verabreichungsweisen enthält.

25. Verwendung nach Anspruch 24, **dadurch gekennzeichnet, dass** die Zusammensetzung für die parenterale Verabreichung angepasst ist.

26. Verwendung nach einem der Ansprüche 1 bis 25, **dadurch gekennzeichnet, dass** die Nukleinsäure eine Desoxyribonukleinsäure ist.

27. Verwendung nach einem der Ansprüche 1 bis 25, **dadurch gekennzeichnet, dass** die Nukleinsäure eine Ribonukleinsäure ist.

28. Verwendung nach einem der Ansprüche 1 bis 27, **dadurch gekennzeichnet, dass** die Nukleinsäure synthetischen oder biosynthetischen Ursprungs ist oder aus einem Virus oder einem prokaryotischen oder eukaryotischen, einzelligen oder mehrzelligen Organismus extrahiert wird.

29. Verwendung nach Anspruch 28, **dadurch gekennzeichnet, dass** die verabreichte Nukleinsäure in Kombination mit der Gesamtheit oder einem Teil der Bestandteile des Herkunftsorganismus und/oder des Synthesesystems vorliegt.

30. Verwendung nach einem der Ansprüche 1 bis 29, **dadurch gekennzeichnet, dass** die Nukleinsäure eine RNA oder ein Protein von Interesse kodiert.

31. Verwendung nach Anspruch 30, **dadurch gekennzeichnet, dass** die RNA eine katalytische oder Antisinn-RNA ist.

32. Verwendung nach Anspruch 30, **dadurch gekennzeichnet, dass** die Nukleinsäure ein Protein kodiert, welches ausgewählt wird aus den Enzymen, den Blutderivaten, den Hormonen, den Lymphokinen, den Zytokinen, den Wachstumsfaktoren, den trophischen Faktoren, den Angiogenese-Faktoren, den neurotrophen Faktoren, den Knochenwachstumsfaktoren, den hämopoetischen Faktoren, den Gerinnungsfaktoren, den Antigenen und den Proteinen, welche an dem Stoffwechsel der Aminosäuren, der Lipide und anderer essentieller Bestandteile der Zelle beteiligt sind.

33. Verwendung nach Anspruch 32, **dadurch gekennzeichnet, dass** die Nukleinsäure die Angiogenese-Faktoren VEGF und FGF, die neurotrophen Faktoren BDNF, CNTF, NGF, IGF, GMF, aFGF, NT3, NT5, das Protein Gax, Wachstumshormon, α-1-Antitrypsin, Calcitonin, Leptin und die Apolipoproteine, die Enzyme der Biosynthese der Vitamine, der Hormone und der Neuromediatoren kodiert.

34. Verwendung nach Anspruch 30, **dadurch gekennzeichnet, dass** die Nukleinsäure einen Antikörper, ein variables Antikörper-Einzelkettenfragment (ScFv) oder ein jegliches anderes Antikörperfragment, welches Erkennungsfähigkeiten in einem Immuntherapie-Kontext aufweist, kodiert oder einen löslichen Rezeptor, ein Agonisten- oder Antagonistenpeptid hinsichtlich eines Rezeptors oder eines Adhäsionsproteins, ein künstliches, chimäres oder verkürztes Protein kodiert.

35. Verwendung nach Anspruch 34, **dadurch gekennzeichnet, dass** die Nukleinsäure einen Antiidiotyp-Antikörper, ein lösliches Fragment des CD4-Rezeptors oder des TNFa-Rezeptors oder des Acetylcholinrezeptors kodiert.

36. Verwendung nach einem der Ansprüche 32 bis 35, **dadurch gekennzeichnet, dass** die Nukleinsäure eine Vorstufe eines therapeutischen Proteins kodiert.

37. Verwendung nach einem der Ansprüche 1 bis 36, **dadurch gekennzeichnet, dass** die Nukleinsäure in Form eines Plasmids vorliegt.

38. Verwendung nach einem der Ansprüche 1 bis 36, **dadurch gekennzeichnet, dass** die Nukleinsäure ein Gen von großer Größe und/oder Introns und/oder regulatorische Elemente von kleiner oder großer Größe enthält.

39. Verwendung nach einem der Ansprüche 1 bis 36, **dadurch gekennzeichnet, dass** die Nukleinsäure eine episomale DNA oder ein künstliches Hefe-Chromosom oder ein Minichromosom ist.

40. Verwendung nach einem der Ansprüche 1 bis 39, **dadurch gekennzeichnet, dass** die Nukleinsäure Sequenzen enthält, welche die Expression des Transgens in dem Gewebe erlauben und/oder begünstigen.

41. Verwendung nach einem der Ansprüche 1 bis 40, **dadurch gekennzeichnet, dass** die Säure in Kombination mit einer jeglichen Art von Vektoren oder mit einer jeglichen Kombination von Vektoren, welche erlauben, den Transfer von Nukleinsäure zu verbessern, wie Viren, synthetischen oder biosynthetischen Mitteln, oder ferner mit zu beschleunigenden oder nicht zu beschleunigenden Kugeln vorliegt.

42. Verwendung nach einem der Ansprüche 1 bis 41, **dadurch gekennzeichnet, dass** das Gewebe einer Behandlung, die darauf abzielt, den Gentransfer zu verbessern, einer Behandlung von pharmakologischer Natur unter örtlicher oder systemischer Anwendung oder einer enzymatischen, permeabilisierenden, chirurgischen, mechanischen, thermischen oder physikalischen Behandlung unterworfen wird.

43. Verwendung nach einem der Ansprüche 1 bis 41, **dadurch gekennzeichnet, dass** sie erlaubt, durch das Gewebe ein Mittel in physiologischen und/oder therapeutischen Dosen entweder in den Muskelzellen, oder welches sekretiert wird, produzieren zu lassen.

44. Verwendung nach einem der Ansprüche 1 bis 43, **dadurch gekennzeichnet, dass** sie erlaubt, die exprimierte Menge von Transgen durch Modulation des Volumens von transfiziertem Gewebe zu modulieren.

45. Verwendung nach Anspruch 44, **dadurch gekennzeichnet, dass** sie erlaubt, das Volumen von transfiziertem Gewebe durch Verwendung einer Mehrzahl von Verabreichungsstellen zu modulieren.

46. Verwendung nach einem der Ansprüche 1 bis 45, **dadurch gekennzeichnet, dass** sie erlaubt, die exprimierte Menge von Transgen zu modulieren, indem die Anzahl, die Form, die Oberfläche und die Anordnung der Elektroden moduliert und indem die Feldstärke, die Anzahl, die Dauer, die Frequenz und die Form der Impulse wie auch die Verabreichungsmenge und das Verabreichungsvolumen der Nukleinsäure variiert werden.

47. Verwendung nach einem der Ansprüche 1 bis 46, **dadurch gekennzeichnet, dass** sie erlaubt, die Lokalisierung der zu transfizierenden Gewebe durch das Gewebevolumen, welches den örtlichen elektrischen Impulsen unterworfen wird, zu kontrollieren.

48. Verwendung nach einem der Ansprüche 1 bis 47, **dadurch gekennzeichnet, dass** sie eine Rückkehr zu der anfänglichen Situation durch operative Entfernung der Zone von transfiziertem Gewebe erlaubt.

49. Nukleinsäure und ein System, welches ein elektrisches Feld mit einer Intensität zwischen 1 und 600 Volt/cm eingeschlossen erzeugt, als Kombinationsprodukt für deren getrennte oder zeitlich gestaffelte Verabreichung *in vivo* an einen Tumor und für die Gentherapie, welche auf der Elektrotransfektion *in vivo* von Geweben nach der Verabreichung der Nukleinsäure beruht.

50. Kombinationsprodukt nach Anspruch 49, **dadurch gekennzeichnet, dass** die Feldstärke zwischen 200 und 600 Volt/cm eingeschlossen liegt.

51. Kombinationsprodukt nach Anspruch 49, **dadurch gekennzeichnet, dass** die Feldstärke ungefähr 500 Volt/cm beträgt.

52. Kombinationsprodukt nach einem der Ansprüche 49 bis 51, **dadurch gekennzeichnet, dass** die gesamte Anwendungsdauer des elektrischen Felds über 10 Millisekunden liegt.

53. Kombinationsprodukt nach einem der Ansprüche 49 bis 52, **dadurch gekennzeichnet, dass** die Anwendung des elektrischen Felds auf das Gewebe einen oder mehrere Impulse von regelmäßiger Frequenz umfasst.

54. Kombinationsprodukt nach Anspruch 53, **dadurch gekennzeichnet, dass** die Anwendung des elektrischen Felds auf das Gewebe zwischen 1 und 100000 Impulse mit einer Frequenz zwischen 0,1 und 1000 Hertz umfasst.

55. Kombinationsprodukt nach einem der Ansprüche 49 bis 52, **dadurch gekennzeichnet, dass** die elektrischen Impulse auf unregelmäßige Weise bezogen aufeinander abgegeben werden und dass die Funktion, welche die Stärke des elektrischen Felds abhängig von dem Zeitpunkt eines Impulses beschreibt, variabel ist.

56. Kombinationsprodukt nach einem der Ansprüche 49 bis 55, **dadurch gekennzeichnet, dass** das Integral der Funktion, welche die Variation des elektrischen Felds mit der Zeit beschreibt, über 1 kV·ms/cm liegt.

57. Kombinationsprodukt nach Anspruch 56, **dadurch gekennzeichnet, dass** dieses Integral größer oder gleich 5 kV·ms/cm beträgt.

58. Kombinationsprodukt nach einem der Ansprüche 49 bis 57, **dadurch gekennzeichnet, dass** die elektrischen Impulse ausgewählt werden aus den Impulsen mit Rechteckwellen, den elektrischen Feldern, welche Wellen mit exponentieller Abnahme, unipolare oszillierende Wellen von begrenzter Dauer, bipolare oszillierende Wellen von begrenzter Dauer oder andere Wellenformen erzeugen.

59. Kombinationsprodukt nach einem der Ansprüche 49 bis 58, **dadurch gekennzeichnet, dass** die elektrischen Impulse Impulse mit Rechteckwellen umfassen.

60. Kombinationsprodukt nach einem der Ansprüche 49 bis 59, **dadurch gekennzeichnet, dass** die elektrischen Impulse äußerlich anzuwenden sind.

61. Kombinationsprodukt nach einem der Ansprüche 49 bis 59, **dadurch gekennzeichnet, dass** die elektrischen Impulse im Inneren des Gewebes anzuwenden sind.

62. Kombinationsprodukt nach einem der Ansprüche 49 bis 61, **dadurch gekennzeichnet, dass** die Nukleinsäuren in das Gewebe zu injizieren ist.

63. Kombinationsprodukt nach einem der Ansprüche 49 bis 61, **dadurch gekennzeichnet, dass** die Nukleinsäure systemisch zu injizieren ist.

64. Kombinationsprodukt nach Anspruch 63, **dadurch gekennzeichnet, dass** die Nukleinsäure auf intraarteriellem oder intravenösem Wege zu injizieren ist.

65. Kombinationsprodukt nach einem der Ansprüche 49 bis 61, **dadurch gekennzeichnet, dass** die Nukleinsäure auf topischem, kutanem, oralem, vaginalem, intranasalem, intramuskulärem, subkutanem oder intraokularem Wege zu verabreichen ist.

66. Kombinationsprodukt nach einem der Ansprüche 49 bis 65, **dadurch gekennzeichnet, dass** die Nukleinsäure in einer Zusammensetzung vorliegt, welche außerdem pharmazeutisch annehmbare Vehikel für die verschiedenen Verabreichungsweisen enthält.

67. Kombinationsprodukt nach Anspruch 66, **dadurch gekennzeichnet, dass** die Zusammensetzung für die parenterale Verabreichung angepasst ist.

68. Kombinationsprodukt nach einem der Ansprüche 49 bis 67, **dadurch gekennzeichnet, dass** die Nukleinsäure eine Desoxyribonukleinsäure ist.

69. Kombinationsprodukt nach einem der Ansprüche 49 bis 67, **dadurch gekennzeichnet, dass** die Nukleinsäure eine Ribonukleinsäure ist.

70. Kombinationsprodukt nach einem der Ansprüche 49 bis 69, **dadurch gekennzeichnet, dass** die Nukleinsäure synthetischen oder biosynthetischen Ursprungs ist oder aus einem Virus oder einem prokaryotischen oder eukaryotischen, einzelligen oder mehrzelligen Organismus extrahiert wird.

71. Kombinationsprodukt nach Anspruch 70, **dadurch gekennzeichnet, dass** die verabreichte Nukleinsäure in Kombination mit der Gesamtheit oder einem Teil der Bestandteile des Herkunftsorganismus und/oder des Synthesesystems vorliegt.

72. Kombinationsprodukt nach einem der Ansprüche 49 bis 71, **dadurch gekennzeichnet, dass** die Nukleinsäure eine RNA oder ein Protein von Interesse kodiert.

73. Kombinationsprodukt nach Anspruch 72, **dadurch gekennzeichnet, dass** die RNA eine katalytische oder Antisinn-RNA ist.

74. Kombinationsprodukt nach Anspruch 72, **dadurch gekennzeichnet, dass** die Nukleinsäure ein Protein kodiert, welches ausgewählt wird aus den Enzymen, den Blutderivaten, den Hormonen, den Lymphokinen, den Wachstumsfaktoren, den trophischen Faktoren, den Angiogenese-Faktoren, den neurotrophen Faktoren, den Knochenwachstumsfaktoren, den hämopoetischen Faktoren, den Gerinnungsfaktoren, den Antigenen und den Proteinen, welche an dem Stoffwechsel der Aminosäuren, der Lipide und anderer essentieller Bestandteile der Zelle beteiligt sind.

75. Kombinationsprodukt nach Anspruch 74, **dadurch gekennzeichnet, dass** die Nukleinsäure die Angiogenese-Faktoren VEGF und FGF, die neurotrophen Faktoren BDNF, CNTF, NGF, IGF, GMF, aFGF, NT3, NT5, das Protein Gax, Wachstumshormon, ein Zytokin, α-1-Antitrypsin, Calcitonin, Leptin und die Apolipoproteine, die Enzyme der Biosynthese der Vitamine, der Hormone und der Neuromediatoren kodiert.

76. Kombinationsprodukt nach Anspruch 72, **dadurch gekennzeichnet, dass** die Nukleinsäure einen Antikörper, ein variables Antikörper-Einzelkettenfragment (ScFv) oder ein jegliches anderes Antikörperfragment, welches Erkennungsfähigkeiten in einem Immuntherapie-Kontext aufweist, kodiert oder einen löslichen Rezeptor, ein Agonisten- oder Antagonistenpeptid hinsichtlich eines Rezeptors oder eines Adhäsionsproteins, ein künstliches, chimäres oder verkürztes Protein kodiert.

77. Kombinationsprodukt nach Anspruch 76, **dadurch gekennzeichnet, dass** die Nukleinsäure einen Antiidiotyp-Antikörper. ein lösliches Fragment des CD4-Rezeptors oder des TNFa-Rezeptors oder des Acetylcholinrezeptors kodiert.

78. Kombinationsprodukt nach einem der Ansprüche 74 bis 77, **dadurch gekennzeichnet, dass** die Nukleinsäure eine Vorstufe eines therapeutischen Proteins kodiert.

79. Kombinationsprodukt nach einem der Ansprüche 49 bis 78, **dadurch gekennzeichnet, dass** die Nukleinsäure in Form eines Plasmids vorliegt.

80. Kombinationsprodukt nach einem der Ansprüche 49 bis 78, **dadurch gekennzeichnet. dass** die Nukleinsäure ein Gen von großer Größe und/oder Introns und/oder regulatorische Elemente von kleiner oder großer Größe enthält.

81. Kombinationsprodukt nach einem der Ansprüche 49 bis 78, **dadurch gekennzeichnet, dass** die Nukleinsäure eine episomale DNA oder ein künstliches Hefe-Chromosom oder ein Minichromosom ist.

82. Kombinationsprodukt nach einem der Ansprüche 49 bis 81, **dadurch gekennzeichnet, dass** die Nukleinsäure Sequenzen enthält, welche die Expression des Transgens in dem Gewebe erlauben und/oder begünstigen.

83. Kombinationsprodukt nach einem der Ansprüche 49 bis 82, **dadurch gekennzeichnet, dass** die Säure in Kombination mit einer jeglichen Art von Vektoren oder einer jeglichen Kombination von Vektoren, welche erlauben, den Transfer von Nukleinsäure zu verbessern, wie Viren, synthetischen oder biosynthetischen Mitteln, oder ferner mit zu beschleunigenden oder nicht zu beschleunigenden Kugeln vorliegt.

84. Kombinationsprodukt nach einem der Ansprüche 49 bis 83, **dadurch gekennzeichnet, dass** das Gewebe einer Behandlung, die darauf abzielt, den Gentransfer zu verbessern, einer Behandlung von pharmakologischer Natur unter örtlicher oder systemischer Anwendung oder einer enzymatischen, permeabilisierenden, chirurgischen, mechanischen, thermischen oder physikalischen Behandlung unterworfen wird.

85. Kombinationsprodukt nach einem der Ansprüche 49 bis 84, **dadurch gekennzeichnet, dass** es erlaubt, durch das Gewebe ein Mittel in physiologischen und/oder therapeutischen Dosen entweder in den Zellen des Gewebes, oder welches sekretiert wird, produzieren zu lassen.

86. Kombinationsprodukt nach einem der Ansprüche 49 bis 84, **dadurch gekennzeichnet, dass** es erlaubt, die exprimierte Menge von Transgen durch Modulation des Volumens von transfiziertem Gewebe zu modulieren.

87. Kombinationsprodukt nach Anspruch 86, **dadurch gekennzeichnet, dass** es erlaubt, das Volumen von transfiziertem Gewebe durch Verwendung einer Mehrzahl von Verabreichungsstellen zu modulieren.

88. Kombinationsprodukt nach einem der Ansprüche 49 bis 87, **dadurch gekennzeichnet, dass** es erlaubt, die exprimierte Menge von Transgen zu modulieren, indem die Anzahl, die Form, die Oberfläche und die Anordnung der Elektroden moduliert und indem die Feldstärke, die Anzahl, die Dauer, die Frequenz und die Form der Impulse wie auch die Verabreichungsmenge und das Verabreichungsvolumen der Nukleinsäure variiert werden.

89. Kombinationsprodukt nach einem der Ansprüche 49 bis 88, **dadurch gekennzeichnet, dass** es erlaubt, die Lokalisierung der transfizierten Gewebe durch das Gewebevolumen, welches den örtlichen elektrischen Impulsen unterworfen wird, zu kontrollieren.

90. Kombinationsprodukt nach einem der Ansprüche 49 bis 89, **dadurch gekennzeichnet, dass** es eine Rückkehr zu der anfänglichen Situation durch operative Entfernung der Zone von transfiziertem Gewebe erlaubt.
